# EUROPEAN PATENT SPECIFICATION

(11) **EP 0 953 634 B1**
(45) Date of publication and mention of the grant of the patent: **07.06.2006**
(21) Application number: 97944193.8
(22) Date of filing: 21.10.1997
(51) Int. Cl.: C12N 9/02, C08G 65/44

(54) **COMPOSITION FOR TREATING POROUS ARTICLE, TREATMENT METHOD, AND USE THEREOF**
ZUSAMMENSETZUNGEN ZUR BEHANDLUNG EINES PORÖSEN GEGENSTANDES, BEHANDLUNGSVERFAHREN UND DESSEN VERWENDUNG
COMPOSITION DE TRAITEMENT D'UN ARTICLE POREUX, PROCEDE DE TRAITEMENT, ET SON UTILISATION

(30) Priority: 06.12.1996 JP 32725296; 30.05.1997 JP 14238697
(43) Date of publication of application: 03.11.1999
(73) Proprietor: SDS Biotech K.K., Tokyo 105-0014 (JP)
(72) Inventor: ECHIGO, Takashi, Showa Denko K.K., Chiba-shi, Chiba 267 (JP); OHNO, Ritsuko, Shoko Co., Ltd., Tokyo 105 (JP)
(74) Representative: Strehl Schübel-Hopf & Partner
(86) International application number: PCT/JP1997/003798
(87) International publication number: WO 1998/024890

(56) References cited:
- EP-A- 0 197 908
- EP-A- 0 919 628
- WO-A-87/02939
- WO-A-95/01426
- WO-A-95/07988
- JP-A- 5 117 591
- JP-A- 6 287 516
- JP-A- 6 316 874
- JP-A- 7 126 354
- JP-A- 7 126 377
- DATABASE WPI Section Ch, Week 198925 Derwent Publications Ltd., London, GB; Class A96, AN 1989-182847 XP002128042 & JP 01 121342 A (OJI PAPER CO), 15 May 1989 (1989-05-15)
- JOURNAL OF BIOTECHNOLOGY, (1990), Vol. 13, RITTER D. et al., "Polymerization and Depolymerization of Lignosulfonate by Phanerochaete Chrysosporium Immobilized on Foam", pages 229-241.

## Description

### TECHNICAL FIELD

This invention relates to a method for treating a porous article such as a sintered metal article, a cast article, an alloy, a die-cast article, a ceramic, a brick, concrete, wood, processed woody material, wood chips, wood powder, chaffs, rush, straw, bamboo, fibers, paper, pulp, foamed synthetic resin, etc., to a composition useful in the treatment, and to use thereof.

More particularly, this invention relates to a method for treating a porous article by impregnating it with an enzyme which oxidizes polyphenols in an alkaline pH range, a phenolic compound and/or an aromatic amine compound, as well or a chemical agent, as defined in the present claims, under pressure or reduced pressure for macromolecularization reaction in the porous article, to a composition for use in the treatment method, and treated products from the porous article obtained by the treatment method which are imparted or increased in strength, wear resistance, weatherability, rust-preventing properties, flame resistance antibacterial properties, antiseptic properties, sterilizing properties, insect-repellent properties, insecticidal properties, antiviral properties, organism-repellent properties, adhesiveness, chemical agent-slow-releasing properties, coloring properties, dimension stability, crack resistance, deodorizing properties, deoxidizing properties, humidity controlling properties, moisture conditioning properties, water repellency, surface smoothness, bioaffinity, ion exchangeability, formaldehyde absorbing properties, chemical agent elution preventing properties, or properties preventing the migration of inorganic compounds onto the surface of the porous article.

### BACKGROUND ART

Hitherto, for modifying physical properties, imparting strength, manufacturing laminated articles, imparting antibacterial or vermin-preventing properties or the like, a thermosetting resin is impregnated or coated to porous materials such as wood, wood chips, wood powder, fibers, paper or pulp and then heated for macromolecularization therein. However, since the thermosetting resin contains unreacted formaldehyde, its influences on human body are feared and, hence, there has been a demand for utilization of compounds that contain no formaldehyde. Also, use of thermosetting resins is disadvantageous since it requires heating at 80 to 200 °C for curing so that special heating appliance and energy for heating are necessary.

Further, various enzyme macromolecularization processes have heretofore been practiced which use as a macromolecularization catalyst polyphenol oxidation enzyme, for example, laccase or polyphenol oxidases produced by Basidiomycetes or Deuteromycetes as disclosed in W087-2939, Japanese Patent Application Kokai Nos. H5-117591 and H6-287516, H7-126354, and H7-126377, and *Journal of Biotechnology,* **13**, 229-241, 1990 and so on. However, these enzymatic macromolecularization methods relate to enzymatic macromolecularization processes in a solution or on a surface of solid and to its applications. Further, the method of applying the macromolecularization reaction of urushiol and the like natural substances that contain no formaldehyde using a laccase as a macromolecularization catalyst in the manufacture of coating compositions or adhesives, has been used as an ancient Japanese lacquer, and is also tried to apply in order to increase the water resistance of a cardboard or corrugated paper by coating a polyphenol oxidizing enzyme, such as laccase, together with its substrate. However, the use of urushi or the like technique essentially utilizes a macromolecularization reaction on the surface of a solid or the lamination surface between solids and the additives are limited to those pigments used for coloring or water-soluble polysaccharides imitating the natural urushi, so that utilization of urushi-like reactions in industry have also been limited.

Furthermore, since the laccase or polyphenol oxidases produced by fungi have optimum reaction pH values in an acidic region, the reaction has heretofore had to be carried out in an acidic to neutral regions in order to promote the macromolecularization reactions with the enzymes, and in addition, the rate of the macromolecularization reaction is not so high. Further, many natural organic compounds on which these enzymes are active are polyphenol compounds, whose solubility decreases in acidic to neutral pH regions while the optimum reaction pH of the enzyme remains in the acidic region. This necessitates that the reaction be carried out in acidic to neutral pH regions, causing a problem that high concentration polyphenol compounds are difficult to be macromolecularized efficiently. For many of the polyphenol compounds, their auto-oxidation has not been utilized effectively since they have been subjected to enzymatic oxidative macromolecularization in acidic to neutral pH regions although their auto-oxidation is accelerated in an alkaline pH region.

It is known that phenolic compounds can also be macromolecularized with bilirubin oxidase and this reaction can be utilized in the macromolecularization of lignin and dyeing of cotton (cf. WO95-01426 and Japanese Patent Application Kokai No. H6-316874). However, even in the case of the prior art technique using bilirubin oxidase, the enzyme-catalystic macromolecularization of phenolic compounds has been practiced in acidic to neutral pH regions, so that the rate of the macromolecularization has not been so high.

Japanese Patent Application Kokai No. S61-268729 discloses a method for impregnating wood including the first step of adding a lignin derivative to wood and the second step of impregnating the wood with a weak acidic aqueous solution containing metal ions for making lignin water-insoluble in order to prevent the attack by microorganisms. However, in this method, the solubility in water of a complex of the lignin derivative with the metal ions decreases while the lignin derivative itself is not fixed as a water-insoluble substance. Further, for the treatment, two kinds of the treating agents have to be used switching one from another, so that it is difficult to apply the method to the existing appliance for injecting CCA (chromium-copper-arsenic type preservative) based aqueous solution which allows one pack treatment and is currently used in the main as a wood preservative. Therefore, it has been desired to develop a method of utilizing a lignin derivative which fixes to wood strongly and which allows one pack treatment.

Furthermore, efforts have been made for impregnating porous articles with various chemical agents to modify their properties but it has been difficult for the porous articles to retain their properties effectively for a long period of time because of elution of the injected chemical agents or there has been the fear that leached chemical agents could cause environmental pollution or could have any adverse influence on human body. Therefore, there has been a strong demand for a method for treating a porous article which has a strong preventive effect for elution of chemical agents, retains the effectiveness of the added chemical agents for a long period of time and allows easy treatment operation.

### OBJECT OF THE INVENTION

Accordingly, an object of this invention is to provide a method for treating a porous article using an enzyme catalyst which macromolecularizes a phenolic compound and/or an aromatic amine compound in a porous article according to present claims 1-23.

Another object of this invention is to provide a method for treating a porous article including the step of macromolecularizing efficiently a phenolic compound and/or an aromatic amine compound in an alkaline pH range by using an enzyme which oxidizes polyphenols, which are given or increased in strength, wear resistance, weatherability, rust-preventing properties, flame resistance, antibacterial properties, antiseptic properties, sterilizing properties, insect-repellent properties, insecticidal properties, antiviral properties, organism-repellent properties, adhesiveness, chemical agent-slow-releasing properties, coloring properties, dimension stability, crack resistance, deodorizing properties, deoxidizing properties, humidity controlling properties, moisture conditioning properties, water repellency, surface smoothness, bioaffinity, ion exchangeability, formaldehyde absorbing properties, chemical agent elution preventing properties, or properties preventing the migration of inorganic compounds onto the surface of the porous article, to provide a treating agent therefor, and to provide a treated product thereby.

### DISCLOSURE OF THE INVENTION

The inventors have made intensive investigation in order to develop a method for imparting porous materials such as wood or the like with, or improving the properties of, long-lasting strength, wear resistance, weatherability, rust-preventing properties, flame resistance, antibacterial properties, antiseptic properties, sterilizing properties, insect-repellent properties, insecticidal properties, antiviral properties, organism-repellent properties, adhesiveness, chemical agent-slow-releasing properties, coloring properties, dimension stability, prevention of cracking, deodorizing properties, deoxidizing properties, humidity controlling properties, moisture conditioning properties, water repellency, surface smoothness, bioaffinity, ion exchangeability, formaldehyde absorbing properties, chemical agent elution preventing properties, or properties preventing the migration of inorganic compounds onto the surface of the porous article. As a result, the inventors have discovered that the objects of this invention can be achieved by impregnating a porous article with an enzyme having an action of oxidizing a polyphenol, and a phenolic compound and/or an aromatic amine compound, and an unsaturated compound or chemical agent, under pressure or reduced pressure, if desired, and allowing macromolecularization reaction in the porous article and completed this invention.

Therefore, this invention uses an enzyme or enzymatic system which has polyphenol oxidizing action utilizing oxygen (air) as an oxidizer. It is surprising that such an enzyme or enzymatic system catalyzes oxidation reaction and macromolecularization reaction in the environment inside the porous article where oxygen is supplied at a low rate. In particular, the impregnation step, which involves a pressure reduction operation as a part thereof, is effective as an operation for increasing the amount of the treating liquid into porous articles which are difficult to impregnate. However, the treating liquid and treated product after the pressure reduction operation contain a decreased concentration of dissolved oxygen in the liquid, which is disadvantageous for the catalyst reaction which utilizes oxygen as an oxidizer. Surprisingly, however, the inventors have discovered that even in the case of the treated porous article after the pressure reduction operation, catalytic oxidation reaction and macromolecularization reaction proceed in the inside thereof. Also, it is known that although impregnation under pressure is effective as an operation for increasing the amount of the treating liquid injected into porous articles which is difficult to impregnate, many enzymes are unstable to pressurization treatment. Surprisingly, again, it has been discovered that polyphenol oxidizing enzyme as a polyphenol oxidizing catalyst retains its catalytic activity even after pressurized injection treatment and catalyzes the oxidation reaction and macromolecularization reaction in the environment inside the porous article, where the oxygen is supplied at a low speed.

In order to increase the treatment effect of porous article or effectively utilizing the chemical agent-fixing or -slow releasing function of the phenolic compound and/or an aromatic amine compound macromolecularized in the porous article, intensive investigation has been made on combined use of various fragrants, deodorants, rust preventives, flame retardants, antibacterial agents, antiseptics, sanitizers, insect-repellents, antiviral agents, or organism-repellents. As a result, it has now been discovered that macromolecularization reaction using an enzyme having a polyphenol oxidizing activity enables fixation and slow release of many chemical agents which generally are considered to cause inhibition of the enzyme reaction or deactivation of the enzyme and this invention has been completed based on the discovery.

That is, this invention provides,a composition for treating a porous article, a treating method and use thereof as described in the present claims.

### BRIEF DESCRIPTION OF THE DRAWINGS

Fig. 1(A) is a perspective view showing a vessel used in examples for performing impregnation treatment under reduced pressure or under pressure;
Fig. 1(B) is a cross-sectional view showing the vessel for impregnation shown in Fig. 1(A); and
Fig. 2 is a perspective showing a box formed by folding commercially available paper towel used in Example 1.9.

### DETAILED DESCRIPTION OF THE INVENTION

Hereinafter, this invention will be described in detail.

### [Polyphenol Oxidizing Enzymes]

The enzyme which is used in this invention for macromolecularization reaction after being coated or impregnated to a porous article may be of any type as far as it has a polyphenol oxidizing activity. Examples of the enzymes include polyphenol oxidizing enzymes such as a catechol oxidase, a laccase, a polyphenol oxidase, an ascorbic acid oxidase, and a bilirubin oxidase, which are produced by microorganisms such as fungi or bacteria, or plants. In particular, when it is desired to practice high rate macromolecularization reaction, those having a polyphenol oxidizing activity in an alkaline pH region are desirable.

Furthermore, the macromolecularization reaction by enzymatic oxidation may be practiced using an enzyme having a peroxidase activity, such as a peroxidase, a lignin peroxidase, a manganese peroxidase or the like from microorganisms or plants, and hydrogen peroxide. The addition or supply of hydrogen peroxide can be achieved by a method in which an aqueous solution of hydrogen peroxide is added directly, a method in which use is made of a hydrogen peroxide precursor such as a perborate, a percarbonate, or the like in place of hydrogen peroxide, or a method in which use is made of an oxidase which can produce hydrogen peroxide and a substrate therefor. However, in order to coat or impregnate the solution to a porous article, which is a target of this invention, for macromolecularization reaction to treat the porous article, it is necessary to increase the permeability of the treating liquid into the porous article so that it is desirable to suppress the macromolecularization reaction before the coating or before during the impregnation treatment.

However, a mixture of hydrogen peroxide or its precursor and the enzyme having a peroxidase activity is difficult to use for the purpose of this invention since oxidation reaction and macromolecularization reaction proceed immediately when it is formulated as a treating liquid. However, use of a mixture of a substance having a peroxidase activity and an oxidase capable of producing hydrogen peroxide, under the conditions where supply of oxygen is cut, oxidation reaction and macromolecularization reaction of polyphenol by the peroxidase will not proceed substantially even when the substrate for the oxidase is present since hydrogen peroxide is not generated in amounts larger than is produced by the oxidase utilizing the dissolved oxygen. Therefore, the object of this invention can also be achieved with a mixture of the enzyme having a peroxidase activity and the oxidase capable of producing hydrogen peroxide.

Examples of the oxidase capable of producing hydrogen peroxide include glucose oxidases, alcohol oxidases, glycerol oxidases, amine oxidases, amino acid oxidases, D-amino acid oxidases, allyl alcohol oxidases, aldehyde oxidases, galactose oxidases, sorbose oxidases, ureate oxidases, xanthine oxidases, cholesterol oxidases, etc. In particular, glucose oxidases, alcohol oxidases are desirable.

The enzyme having a polyphenol oxidizing activity in an alkaline pH region which can be used in this invention may be desirably the one which has an optimum reaction pH for the polyphenol oxidation reaction on an alkaline pH side, e.g., pH 7.5 or higher in order to practice efficient macromolecularization reaction. More specifically, such an enzyme is desirably the one having an optimum reaction pH on an alkaline side, i.e., pH 7.5 or higher as measured by the activity measurement using syringaldazine as described hereinbelow. Further, the enzyme having a peroxidase activity and the oxidase capable of producing hydrogen peroxide which can be used in this invention are desirably those having respective optimum reaction pH values on an alkaline side, i.e., pH 7.5 or higher, in order to practice efficient macromolecularization reaction.

Examples of the microorganisms which produce the enzymes used for the purpose of this invention include the followings.

Examples of Eumycetes fungi include those belonging to the subdivision *Deuteromycotina,* such as *Aspergillus, Botrytis, Myrothecium, Embellisia, Dreschlera, Penicillium, Pestalotia, Rhizoctonia, Tricoderma, Arthromyces, Humicola, Verticillum, Ulocladium, Caldariomyces, Stilbella, Sagenomella,* and *Stachylidium,* and preferably those strains beloging to *Aspergillus nidulans, Botrytis cinerea, Myrothecium roridum, Myrothecium verrucaria, Myrothecium prestonii, Myrothecium leucotrichum, Embellisia alli, Dreschlera halodes, Penicillium sclerotiorum, Penicillium janthinellum, Pestalotia palmarum, Rhizoctonia praticola, Rhizoctonia solani, Tricoderma resii, Tricoderma viride, Arthromyces ramosus, Humicola insolens, Verticillum dahlie, Verticillum alboatrum, Ulocladium chartarum, Caldariomyces fumago, Stilbella annulata, Stilbella bulbicola, Stilbella erythrocephala, Stilbella flavescens, Stilbella flavipes, Stilbella thermophila, Stilbella sp., Sagenomella viride, Sagenomella sp., Stachylidium bicolor, Stachylidium theobromae,* and *Stachylidium sp..* Among these, the most preferred are *Myrothecium verrucaria* SD3001 (deposited under Accession No. FERM P-14955 at Bioengineering and Industrial Technology Research Laboratories, Institute of Science and Industrial Technology, Ministry of International Trade and Industry, Japan, transferred to International Deposition under Accession No. FERM BP-5520) , *Myrothecium verrucaria* IFO *6113, Myrothecium roridum* SD3002 (deposited under Accession No. FERM P-15255 at Bioengineering and Industrial Technology Research Laboratories, Institute of Science and Industrial Technology, Ministry of International Trade and Industry, Japan, transferred to International Deposition under Accession No. FERM BP-5523).

Other preferred Eumycetes fungi include those belonging to the subdivision *Basidiomycotina,* such as *Pleurotus, Lentinus, Schizophyllum, Armillariella, Flammulina, Agaricus, Coprinus, Phanerochaete, Phlebia, Hygrophoropsis, Lenzites, Melanoleuca, Pholiota, Stereumu, Polyporus, Polyporellus, Crucibulum, Microporus, Fomitopsis, Pycnoporus, Collybia, Trametes, Coriolus, Daedaleopsis, Rigidoporus, Fomes, Ganoderma, Trachyderma, Hymenochaete, Inonotus,* and *Psathyrella,* and among these preferred strains are those belonging to *Pleurotus cornucopiae, Pleurotus osteratus, Lentinus edodes, Schizophyllum commune, Armillariella mellea, Flammulina velutipes, Agaricus bisporus, Coprinus comatus, Coprinus cinereus, Coprinus congregatus, Coprinus plicatilis, Coprinus macrorhizus, Phanerochaete chrysosporium, Phlebia radiata, Hygrophoropsis aurantiaca, Lenzites betulina, Melanoleuca verrucipes, Pholiota nameko, Stereumu hirsutum, Polyporus squamosus, Polyporus pinsitus, Polyporellus ba dius, Crucibulum laeve, Microporus flabelliformis, Fomitopsis pinicola, Pycnoporus coccineus, Collybia acervata, Collybia maculata, Trametes orientalis, Trametes villosa, Coriolus versicolor, Coriolus hirsutus, Daedaleopsis tricolor, Rigidoporus zonalis, Fomes fomentarius, Ganoderma lucidum, Trachyderma tsunodae, Hymenochaete rubiginosa, Inonotus mikadoi, Psathyrella multissima,* and *Psathyrella piluliformis.*

Preferred Eumycetes fungi other than those belonging to the subdivisions *Deuteromycotina* and *Basidiomycotina* include those strains belonging to the genera *Podospora, Neurospora, Monocillium,* and *Fusarium* beloging to the subdivision *Ascomycotina,* and those strains belonging to the genera *Mucor, Ascomycotina, Rhizopus* belonging to the subdivision *Zygomycotina,* preferably *Podospora anserina, Neurospora crassa, Monocillium indicum, Fusarium oxysporum, Mucor hiemalis,* and *Rhizopus nigricans.*

Some preferred bacteria include those strains belonging to the genera *Azospirillum,* preferably *Azospirillum lipoferum,* or order *Actinomycetales,* for example, *Streptomyces,* preferably *Streptomyces antibioticus, Streptomyces spheroides, Streptomyces thermoviolaceus,* or *Aerobacter*, preferably *Aerobacter aerogenes.*

Other preferred bacteria include *Bacillus alcalophilus, Bacillus amyloliquefaciens, Bacillus brevis, Bacillus firmus, Bacillus licheniformis, Bacillus subtilis, Bacillus natto, Bacillus pumilus, Bacillus sphaericus,* and *Bacillus stearothermophilus,* and preferably *Bacillus licheniformis.*

Some preferred plants containing the enzymes used in this invention include *Acerpseudoplatanum, Dioscorea, Abelmoschus, Psidium, Helianthus,* potato, apple, pumpkin, cucumber, wheat, soybean, alfalfa, horse radish and the like.

### [Preparation of Enzymes]

The enzymes used in this invention can be obtained by cultivating strains belonging to the above described microorganisms, for example, fungi or bacteria, and variants thereof. Besides, they can also be prepared by utilizing genetically engineered microorganisms. That is, the enzymes can be prepared by cultivating transformed host cells under conditions allowing expression of the enzyme protein and recovering the enzyme protein from the culture medium; the transformed host cells may be obtained by transforming the host cells with an expression vector obtained by inserting in a DNA vector having replication initiation codon a DNA sequence encoding the protein of the above-described enzyme together with the DNA sequences of suitable promoter, operator and terminator having an enzyme expressing function in the host organism, or by incorporating in the host cell DNA, a DNA sequence encoding the protein of the above-described enzyme together with suitable promoter, operator and terminator DNA sequences having an enzyme expressing function in the host cells.

The DNA fragment encoding the enzyme protein used in this invention can be obtained by a conventional method, for example, by identifying the target DNA fragment using the cDNA or genome library from a strain belonging to the above-described microorganisms, for example, a fungus or a bacterium, as an isolation source, and using an oligonucleotide as a probe which is synthesized based on the amino acid sequence of the enzyme protein used in this invention, by screening the clone expressing the activity of an oxidase, or by screening the clone producing a protein which reacts with an antibody of the above-described enzyme protein.

The enzyme protein used in this invention can also be prepared by extraction from seeds, fruits, leaves, etc. of the above-described plants.

Further, in the cultivation of the strains belonging to fungi or bacteria and variants thereof used for obtaining the enzyme proteins used in this invention, there can be used a synthetic medium or a nutrient medium containing organic carbon sources and organic nitrogen sources which are used conventionally. During cultivation, it is desirable to add Cu²⁺ ion as a metal salt in concentrations of 0.001 mM to 10 mM, and preferably 0.01 mM to 1 mM.

In the case where the polyphenol oxidizing enzymes used in this invention are secreted outside the cells of the fungi or bacteria, the enzymes can be recovered from the culture medium by a well-known method. The recovery procedure includes a series of operations such as removing cells from the culture medium by centrifugation, filtration or membrane separation, and employing chromatography, for example, ion exchange chromatography. Also, transmembrane concentration using an ultrafilter membrane is effectively employed. When the enzyme proteins are accumulated in the cells of fungi or bacteria or when they exist in plant tissues, they can be recovered by a well-known method. The recovery procedure includes a series of operations such as mechanical rupture of the tissue by homogenization, separation and extraction of the solution containing the enzyme proteins by centrifugation, filtration or membrane separation, and chromatography, for example, ion exchange chromatography. Transmembrane concentration using an ultrafilter membrane is also effectively employed.

### [Measurement of Activity]

In this invention, the polyphenol oxidation activity of the enzyme protein having a polyphenol oxidizing activity was determined by performing the reaction in an aqueous solution containing 20 ppm of syringaldazine and 100 mM Tris-HCl buffer or potassium phosphate buffer at 20 °C and at the optimum reaction pH and measuring absorbance at 525 nm. The amount of activity in which 1 µmol/minute of syringaldazine is oxidized was defined as 1 unit (hereafter, abbreviated as "U").

The polyphenol oxidation activity of the enzyme having a peroxidase activity was determined by performing the reaction in an aqueous solution containing 20 ppm of syringaldazine, 20 ppm of hydrogen peroxide, and 100 mM Tris-HCl buffer or potassium phosphate buffer at 20 °C and at the optimum reaction pH and measuring absorbance at 525 nm. The amount of activity in which 1 µmol/minute of syringaldazine is oxidized was defined as 1 unit (hereafter, abbreviated as "U").

Furthermore, the oxidase activity producing hydrogen peroxide was determined by performing the reaction in an aqueous solution containing 1 mM to 100 mM of substrate for oxidase, 20 ppm of syringaldazine, 1 U/ml of peroxidase and 100 mM Tris-HCl buffer or potassium phosphate buffer at 20 °C and at the optimum reaction pH and measuring absorbance at 525 nm. The amount of activity in which 1 µmol/minute of syringaldazine is oxidized was defined as 1 unit (hereafter, abbreviated as "U").

### [Phenolic Compounds and Aromatic Amine Compounds]

The phenolic compounds and aromatic amine compounds, i.e., the targets of macromolecularization in this invention, may be any compound as far as the enzyme used in this invention can oxidize that.

Specific examples of such phenolic compounds or aromatic amine compounds include lignin, lignosulfonic acid, humic acid, nitrohumic acid, tannin, catechin, gallic acid, urushiol, 4-hydroxycinnamyl alcohol, o-cumaric acid, p-cumaric acid, coniferyl alcohol, coniferyl aldehyde, ferulic acid, etyl-3,4-dihydroxycinnamic acid, 3-hydroxy-4-methoxycinnamic acid, 3,4-dihydroxycinnamic acid, 3-hydroxy-4-methoxycinnamaldehyde, vanillin, o-vanillin, vanillic acid, vanillyl alcohol, o-vanillyl alcohol, isovanillyl alcohol, vanillylamine, vanillinazine, 4-hydroxy-3-methoxybenzonitrile, syringinic acid, sinapyl alcohol, sinapic acid, sinapinaldehyde, homovanillic acid, homovanillyl alcohol, homovanillonitrile, hesperidin, chlorogenic acid, hinokitiol, pyrocatechol, hydroquinone, tert-butylhydroquinone, phenylhydroquinone, trimethylhydroquinone, pyrogallol, lauryl gallate, octyl gallate, 3,4-dihydroxybenzoic acid, 1,2-dihydroxynaphthalene, 2,3-dihydroxynaphthalene, 6,7-dihydroxy-2-naphthalenesulfonic acid, anthrarobin, alizarin, quinizarin, o-phenylenediamine, p-phenylenediamine, 3,4-diaminobenzophenone, o-anisidine, p-anisidine, o-aminophenol, p-aminophenol, 1,2-diaminoanthraquinone, 1,4-diaminoanthraquinone, and the like compounds, and derivatives thereof.

Other compounds than these compounds may also be used as a raw material for macromolecules or macromolecularization initiator as far as they can be oxidized by the enzymes used in this invention. Examples of such compounds include ABTS (2,2'-azobis(3-ehtylbenzothiazoline-6-sulfonic acid)), bilirubin, ascorbic acid, isoascorbic acid, quercetin, rutin, guaiacol, o-hydroxybenzoic acid, p-hydroxybenzoic acid, 4-methoxyphenol, biphenol, 4,4'-ethylenedianiline, methylhydroquinone, ethylhydroquinone, 1-hydroxybenzotriazole, 6-hydroxy-2,4,5-triaminopyrimidine, 4,5,6-triaminopyrimidine, 2,3-dihydroxypyridazine, 3,6-dihydroxypyridazine, 2,3-dihydroxypyridine, methyl-4-hydroxy-3-methoxybenzoic acid, 4,5-diamino-6-hydroxy-2-mercaptopyrimidine, 2,3-diaminopyridine, 2,5-dihydroxy-1,4-benzoquinone, 2,5-dihydroxybenzoic acid, 3,4-dihydroxy-3-cyclobuten-1,2-dione, 3-(3,4-dihydroxyphenyl)-L-alanine, 2-amino-3-hydroxypyridine, 3-amino-2-methoxybenzofuran, 2,4-dimethoxyaniline, 2,5-dimethoxyaniline, 3,4-dimethoxyaniline, 2',5'-dimethoxyacetophenone, 3',4'-dimethoxyacetophenone, 1,4-dimethoxybenzene, veratrol, 2,3-dimethoxybenzoic acid, 2,5-dimethoxybenzoic acid, veratric acid, veratryl aldehyde, veratrylamine, homoveratric acid, homoveratrylamine, homoveratronitrile, 3,4-dimethoxycinnamic acid, 3,4-dimethoxycinnamonitrile, 2,3-dimethoxyphenol, 3,4-dimethoxyphenol, 3,4-dimethoxybenzyl alcohol, 3,4-dimethoxyphenethylamine, 3,4-dimethoxystyrene, (3,4-dimethoxyphenyl)acetic acid, (3,4-dimethoxyphenyl)-acetonitrile, (3,4-dimethoxyphenyl)acetone, 3-(3,4-dimethoxyphenyl)propionic acid, 3-(3,4-dimethoxyphenyl)propanol, 4-(3,4-dimethoxyphenyl)butyric acid, 3-(3,4-dimethoxyphenyl)propanol, 2-methoxy-4-prophenylphenol, 2-methoxy-5-methylaniline, 2-methoxy-5-nitroaniline, 4-methoxy-2-nitroaniline, 3-methoxysalicylic acid, acetylsalicylic acid, methyl salicylate, ethyl salicylate, 3-methylcatechol, 4-methylcatechol, methyl gallate, propyl gallate, 3,4,5-trimethoxyaniline, 3,4,5-trimethoxyphenol, tropolone, purpurogallin, salicylaldoxime, 3-amino-5,6,7,8-tetrahydro-2-naphthol, 1,5-dihydroxynaphthalene, 3,5-dihydroxy-2-naphthoic acid, 4-hydroxy-1-naphthalenesulfonic acid, purpurin, 2,3-dihydro-9,10-dihydroxy-1,4-anthracenedione, various azo dyes, and derivatives of these compounds.

In order to control the physical properties of the macromolecules, two or more of these phenolic compounds or aromatic amine compounds may be used in combination.

Also, when the macromolecularized phenolic compound or aromatic amine compound is prepared according to this invention, there may be coexist one or more quinone compounds which can be macromolecularized in a similar reaction path. Examples of such quinone compounds include anthraquinone-2-sulfonic acid, anthraquinone-1,5-disulfonic acid, anthraquinone-2,6-disulfonic acid, anthraquinone-2-carboxylic acid, 1-aminoanthraquinone, 2-aminoanthraquinone, anthrarufin, aminonaphthoquinone, 1,8-dihydroxyanthraquinone, camphorquinone, dehydroascorbic acid, 2-hydroxy-1,4-naphthoquinone, isatin, 5-nitroisatin, and various anthraquinone dyes. Further, air oxidation and macromolecularization may be performed simultaneously with the enzymatic reaction in copresence of one or more substances which are susceptible to autooxidation such as unsaturated fatty acids, e.g., oleic acid, rinolic acid, etc., unsaturated alcohols, e.g., oleyl alcohol, etc., unsaturated alkyls, e.g., squalene, etc., drying oils, e.g., tung oil, linseed oil, castor oil, etc. Also, there can be used aromatic compounds having an unsaturated side chain, such as cinnamic acid, cinnamaldehyde, cinnamonitrile, cinnamyl alcohol, cinnamyl acetate, and derivatives thereof.

### [Macromolecularization Reaction Method and Application Thereof]

In the treatment of porous articles such as a sintered metal article, a cast article, an alloy, a die-cast article, a ceramic, a brick, concrete, wood, processed woody material, chaffs, rush, straw, bamboo, foamed synthetic resin, etc., obtained by the method of this invention, it is desirable that when the enzyme having a polyphenol oxidizing activity and the phenolic compound and/or aromatic amine compound are impregnated for macromolecularization reaction in the porous article, the treating liquid is not gelled or solidified before the impregnation. And it is desirable that, after the impregnation, gelation or solidification of the treating liquid proceeds as the treating liquid is dried and concentrated in the porous article. For this purpose, the phenolic compounds and/or aromatic amine compounds are or is in concentrations of 0.01 to 50% by weight, preferably 0.1 to 30% by weight, in the solution during the treatment of the porous article. The reaction temperature is 0 to 100 °C, preferably 10 to 70 °C. Further, the reaction pH is 7.0 to 12, preferably 7.5 to 10. The enzyme activity concentration is 1 to 10,000 U/liter, preferably 10 to 2,000 U/liter. It is desirable that the enzyme activity concentration be adjusted depending on the purpose. That is, when more speedy macromolecularization is attempted, the reaction will be carried out at higher activity concentrations. On the other hand, when the reaction is performed at lower activity concentrations, macromolecularization reaction will proceed more mildly, thus giving rise to a more uniform complex of a macromolecule and the porous article. As soon as an appropriate degree of macromolecularization is reached, the reaction can be termminated by impregnation with an alkali or alkaline salt such as NaOH, NH₃, Na₂CO₃, CaCO₃, or the like, by impregnation with an acid such as hydrochloric acid, sulfuric acid, nitric acid, phosphoric acid, carbonic acid, boric acid, an organic acid, or the like, by impregnation with a known enzyme inhibitor, by heat treatment such as that at 100 °C for 15 minutes, or by cutting off the supply of oxygen by coating the surface of the porous article or wrapping the porous article with a film.

For increasing the effect of enzymatic macromolecularization in the porous article, the enzyme having a polyphenol oxidizing activity and phenolic compound and/or aromatic amine compound together with an unsaturated compound such as an unsaturated fatty acid, an unsaturated alcohol, an unsaturated alkyl compound, or a drying oil are impregnated in a porous article under pressure or under reduced pressure and macromolecularization reaction is allowed to proceed in the porous article so that the macromolecularization reaction of the phenolic compound and/or aromatic amine compound by an enzyme or autooxidation and the macromolecularization reaction of the unsaturated compound based on autooxidation can be performed simultaneously, thus making it possible to obtain stronger complexes. Therefore, this invention is very useful. For this purpose, the unsaturated compound is used in concentrations of 0.001 to 60% by weight, preferably 0.01 to 40% by weight, in the solution during the treatment of the porous article.

This invention is very useful since impregnation of the enzyme having a polyphenol oxidizing activity in a porous article under pressure or under reduced pressure and macromolecularization, in the porous article, of the polyphenol compound and/or aromatic amine compound already contained in the porous article, as the polyphenol compound such as lignin contained in the wood, make it possible, when the porous article is wood, to improve the workability in the drying step after the impregnation treatment of wood, to improve strength of wood which has decreased due to lignin decomposition by wood boiling treatment or high temperature steam injection treatment, to improve the effect of preventing wood cracking upon drying or freezing, or to suppress the growth of microorganisms because the anaerobic environment in the wood is maintained or improved.

Further, this invention is very useful in that impregnation of a porous article with an enzyme having a polyphenol oxidizing activity and a phenolic compound and/or aromatic amine compound in combination under pressure or under reduced pressure and macromolecularization reaction in the porous article enables effective treatment of even such a porous article containing no or a small amount of the substance on which the enzyme having a polyphenol oxidizing activity acts, and that since the enzymatic macromolecularization reaction proceeds mainly in the inside of the porous article, a large amount of treatment liquid can be impregnated with ease by using the treatment liquid in a state where the substance constituting the reaction composition has a low molecular weight and, hence, a relatively low viscosity. In particular, when such a substance as lignin on which the enzyme having a polyphenol oxidizing activity can act is already present in or fixed to the porous article, it is desirable because the substance reacts with the phenolic compound and/or aromatic amine compound by the action of the enzyme in the porous article and macromolecularize, and as a result, the macromolecule produced from the phenolic compound and/or aromatic amine compound is fixed in the porous article more firmly.

Lignin, lignosulfonic acid, or lignosulfonate as is as obtained from the process of manufacturing alkali digested pulp or sulfite pulp, contains various water-insoluble solid components. Therefore, when a porous article is impregnated with an enzyme having a polyphenol oxidizing activity and lignin, lignosulfonic acid or lignosulfonate for macromolecularization reaction in the porous article, it is desirable to remove the water-insoluble solid components in these pulp waste liquors in order to increase the amount of the treatment liquid impregnated into the porous article. It is desirable to practice the removal treatment by a suitable method selected from centrifugation, filtration, standing still and the like depending on the kind of porous article subjected to impregnation treatment, the purpose of impregnation treatment, and the costs involved in the removal. For example, for the purpose of performing pressurized injection treatment to wood, it is desirable to remove water-insoluble solid components in the pulp waste liquor, having a size of at least 1 µm, preferably at least 0.5 µm, more preferably at least 0.1 µm, in diameter or longer diameter.

It is also possible to use a pulp waste liquor from which salts or sugars are removed by ultrafiltration, or fractions having lower molecular weights, e.g., 5,000 to 100,000, in order to increase the amount of impregnated solultion. Removal of sugars can also be practiced by using microorganisms such as yeast. The lignin derivatives used in this invention include besides lignosulfonic acid, acetic acid ester, propionic acid ester, carboxy methyl ether, 2-hydroxyehtyl ether, 2-acetoxyethyl ether or 2-hydroxypropyl ether of lignin or lignosulfonic acid, or those alkylated with a halogenated alkyl or the like, those hydroxymethylated with formalin, those crosslinked with formalin, epoxy compounds, isocyanate compounds, allyl compounds, acetone or the like, or those obtained by crosslinking lignin or lignosulfonic acid together with other phenolic compounds, polyphenol compounds, aromatic amine compounds or the like, those further sulfonated with neutral sulfite solution or the like, those desulfonated by heating or the like treatment, and those obtained by hydrolyzing the lignin or lignin derivatives. Mixtures of these may also be used.

Among the phenolic compounds and/or aromatic amine compounds used in this invention, natural substances such as lignin, lignosulfonic acid, humic acid, nitrohumic acid, tannin, catechin, gallic acid, urushiol, hesperidin, hinokitiol or natural derivatives thereof are highly useful because they are highly safe to environment as well as to human body.

It is very effective to coat or impregnate chemical agents to a porous article as a pretreatment or posttreatment of the macromolecularization treatment of the porous article. In particular, the method is very useful for treating a porous article, comprising a first step of coating or impregnating a chemical agent to a porous article and a second step of impregnating both an enzyme having a polyphenol oxidizing activity and a phenolic compound and/or an aromatic amine compound in the porous article under pressure or under reduced pressure, for the purpose of sequestering the chemical agents in the porous article, particularly preventing the migration of inorganic compounds onto the surface of the porous article. To the contrary, the method is also very useful as an effective treating method using a chemical agent for treating a porous article, comprising a first step of impregnating in a porous article both an enzyme having a polyphenol oxidizing activity and a phenolic compound and/or an aromatic amine compound under pressure or under reduced pressure and a second step of coating or impregnating a chemical agent to the porous article in order to fix the chemical agent to the inside of the porous article utilizing the interaction between the chemical agent and the phenolic compound and/or the aromatic amine compound. By combining with such a pretreatment or posttreatment, the treatment method of this invention, as compared with the case where the porous article is impregnated with a phenolic compound and/or an aromatic amine compound alone, enables the chemical agent to be fixed to the porous article more firmly because the phenolic compound and/or the aromatic amine compound is macromolecularized and fixed to the porous article by enzyme catalytic reaction.

Further, it is possible to control the degree of retaining the porosity of the porous article easily by performing pressure reduction treatment after impregnating the porous article with the treatment liquid to recover a portion of the treatment liquid out of the porous article, or by washing the porous article with water or the like before the macromolecularization reaction in the inside of the treated porous article proceeds sufficiently in order to remove unmacromolecularized compounds. Such a treated product having the thus retained and controlled porosity retains humidity controlling ability, water retention, adsorbing ability, and ion exchange ability, and can be used for various applications utilizing such abilities. Further, chemical agents, macromolecules, pre-macromolecules, and the like can be impregnated to the treated product having a retained porosity to produce porous articles having various composite properties.

The pressurization or pressure reduction performed for the purpose of this invention is important for injecting a sufficient amount of treatment liquid into various types of porous articles which are difficult to impregnate with the treatment liquid so that necessary treatment effects can be obtained. Pressurization operation is performed in the range of 1 atm, which is atmospheric, to 20 atm, preferably 3 to 15 atm. However, a greater pressure may be applied unless the enzyme loses its activity. Pressure reduction operation may be practiced at any pressure until fully vacuumized. For effectively treating porous articles which are difficult to impregnate, a reduced pressure in the ranges of 100 to 760 mmHg is desirable. The pressure reduction operation is preferably of initial vacuum type in which pressure reduction is performed before the treatment liquid is added to a porous article. In order to impregnate a larger amount of treatment liquid, it is also effective that the pressurization operation and the pressure reduction operation be practiced in combination.

In the case where the porous article is wood, various pressurization and pressure reduction treatment methods usually used may be employed. More specifically, a full cell method (Bethell method), a semi-empty cell method (Lowry method), an empty cell method (Reuping method), a double vacuum method, an oscillating pressure method, a pulsation pressure method, a constant pressure method, a slow pressure change method and methods combining these operations are applicable. An incising processing method may also be used in order to increase the amount of impregnated liquid. As a pretreatment for porous articles which are difficult to impregnate, it is also effective to perform compression treatment using a roller or the like, micro wave heating, a freezing treatment, a boiling treatment, a steam treatment, or a heat treatment. Originally, lignin is known to be contained mainly in the heart wood, thus giving an increased resistance to wood-decaying fungi or termites. Accordingly, when especially lignin or lignin derivatives are used as the phenolic compound and/or aromatic amine compound serving as a wood preservative, the treatment method of this invention enables efficient practice of prevention of wood-decaying fungi or termites which is essentially conducted by living wood in the nature as an industrial treatment method applicable to all kinds of wood.

In this invention, for increasing the effect of treatment of a porous article or for effectively utilizing the ability of fixing chemical agents or slowly releasing chemical agents by the phenolic compound and/or aromatic amine compound macromolecularized in the porous article, treatments with various fragrants, deodorants, rust preventives, flame retardants, antibacterial agents, antiseptics, sanitizers, insect-repellents, antiviral agents, or organism-repellents may be practiced as a pretreatment, a simultaneous treatment or a posttreatment. The chemical agents used for the purpose include many existing chemical agents. The chemical agents which can be used are not only water-soluble chemical agents but also those chemical agents which can form O/W type or W/O type emulsions by addition of a dispersant or a surfactant or those chemical agents dispersed in an aqueous solution as fine powder.

Examples of the surfactant used for this purpose include aliphatic sulfates that have straight or branched chain alkyl or alkenyl sulfates, amidosulfates, or straight or branched chain alkyl or alkenyl group, such as alkyls or alkenyl ether sulfates that have one or more of ethylene oxide, propylene oxide and butylene oxide components adducted thereto, aliphatic sulfonates such as alkylsulfonates, amidosulfonates, dialkylsulfo-succicnates, respective sulonates of α-olefins, vinylidene type olefins and inner olefins, aromatic sulfonates such as straight or branched chain alkylbenzensulfonates, alkyl or alkenyl ether carboxylates or carboxamides having a straight or branched chain alkyl or alkenyl group and one or more of ethylene oxide, propylene oxide or butylene oxide components adducted thereto, α-sulfofatty acid salts or esters, amino acid type surfactants, alkyl or alkenyl acidic phosphate esters, phosphate ester type surfactants such as alkyl or alkenyl phosphates, sulfonic acid type amphoteric surfactants, betaine type amphoteric surfactants, alkyl, alkenyl ethers, or alcohols having a straight or branched chain alkyl or alkenyl group and one or more of ethylene oxide, propylene oxide and butylene oxide components adducted thereto, polyoxyethylene alkyl phenyl ethers having a straight or branched chain alkyl or alkenyl group and one or more of ethylene oxide, propylene oxide and butylene oxide components adducted thereto, higher fatty acid alkanolamide or alkylene oxide adducts thereof, sucrose fatty acid esters, fatty acid glycerin monoesters, alkyl or alkenylamine oxides, tetraalkylammonium salt type cationic surfactants, and the like. Many of the known dispersants may be used, and in particular lignin, lignosulfonic acid, or lignosulfonates are useful because they are not only raw materials for macromolecularization reaction by the enzymes having a polyphenol oxidizing activity but also have themselves chemical agent dispersing effects.

Among the above-described chemical agents used in this invention, there can be used as an antibacterial agent, an antiseptic, a sanitizer, an insect-repellent, an antiviral agent, or an organism-repellent, solutions or fine powder of salts, compounds or complexes of the metal e.g., copper, arsenic, zinc, chromium, nickel, aluminum, molybdenum, magnesium, silver. More specifically, metal salts whose anionic moiety is constituted by F⁻, Cl⁻, Br⁻, I⁻, NO₃⁻, BO₃³⁻, PO₄³⁻, P₂O₇⁴⁻, SO₄²⁻, SO₃²⁻, S₂O₃²⁻, SCN⁻, CO₃²⁻, OH⁻, B₄O₇²⁻, or BF⁴⁻, compounds of carboxylic acid such as naphthenic acid, oleic acid, stearic acid, octanoic acid, acetic acid, citric acid, lactic acid, tartaric acid or the like or sulfamic acid with a metal ion, metal oxides, metal oxide ions, or complexes of these, and further hydrates thereof. In addition, there may also be used calcium bromide, sodium bromide, magnesium bromide, potassium bromide, sodium iodide, sodium fluoride, potassium fluoride, sodium fluorosilicate, magnesium fluorosilicate, sodium sulfide, potassium sulfide, potassium selenate, and the like.

As the compounds to be added for forming complexes with the metals, many known compounds can be used. For example, phenolic compounds or aromatic amine compounds such as pyrocatechol, gallic acid, hinokitiol, catechin, pyrogallol, o-phenylenediamine, and 2-aminophenol, phosphonic acids such as ethane-1,1-diphosphonic acid and derivatives thereof, ethanehydroxy-1,1,2-triphosphonic acid, ethane-1,2-dicarboxy-1,2-diphosphonic acid, and methanehydroxyphosphonic acid, phosphonocarboxylic acids such as 2-phosphonobutane-1,2-dicarboxylic acid, 1-phosphonobutane-2,3,4-tricarboxylic acid, and α-methylphosphonosuccinic acid, amino acids or amino acid analogues such as aspartic acid, glutamic acid, glycine, 2-aminoisobutyric acid, and β-alanine, aminopolyacetic acids such as iminodiacetic acid, nitrilotriacetic acid, ethylenediaminetetraacetic acid, and diethylenetriaminepentaacetic acid, high molecular electrolytes such as polyacrylic acid, polyitaconic acid, polymaleic acid, maleic anhydride copolymers, and carboxymethylcellulose, nondissociating polymers such as polyethylene glycol, polyethylene oxide, and polyvinyl alcohol, organic acids such as benzenepolycarboxylic acid, oxalic acid, malic acid, diglycolic acid, succinic acid, oxydisuccinic acid, carboxymethyloxysuccinic acid, gluconic acid, citric acid, lactic acid, tartaric acid, adipic acid, and naphthenic acid, carboxymethylated products of sugars such as sucrose, and lactose, carboxymethylated product of polyhydric alcohols such as pentaerythritol, organic alkali agents such as ethylenediamine, ethanolamine, diethanolamine, triethanolamine, N-methyldiethanolamine, N-ethyldiethanolamine, triethylenetetramine, propanolamine, pentaethylenehexamine, polyethyleneimine, triisopropanolamine, and polyallylamine, cyclic nitrogen-containing compounds such as triazacyclononane and triazacyclododecane or N-methylated derivative thereof, phthalocyanine or porphyrin and derivatives thereof having a hydrophilic substituent group, organic substances such as starch, urea, chitosan, and ξ-polylysine.

The above-described hinokitiol can be used as complexes or salts of various metals and more specifically include complexes with copper, arsenic, zinc, chromium, nickel, aluminum, molybdenum, magnesium, calcium, barium, iron or silver, or sodium salt. In particular, complexes with copper, arsenic, zinc, chromium, nickel or silver are useful because the effect of hinokitiol and that of metal are combined. As the metal powder, metal powder constituted by fine metal particles having various sizes can be used depending on the purpose. For example, when wood is subjected to impregnation treatment, there can be used powder constituted by fine particles having a diameter of 5 µm or less, preferably 0.5 µm or less, and more preferably 0.1 µm or less.

The treatment of porous articles with the metal salts, metal compounds or metal complexes can be practiced as a pretreatment, posttreatment or simultaneous treatment before, after or together with the treatment of the porous article with the polyphenol oxidizing enzyme, or by various methods or by combining them in various manners, depending on the degree of inhibiting the macromolecularization reaction by the polyphenol oxidizing enzyme, solubility under enzyme reaction conditions, occurrence or absence of aggregation or sedimentation when mixed with the treatment agent, purpose of the treatment, etc. The concentration of the metal salts, metal compounds or metal complexes in the treatment liquid is desirably adjusted depending on the intensity of the biological activity the metal to be used has and the purpose of the treatment. For example, in the case of copper, arsenic acid, or zinc, their concentration is usually 0.01 to 500 mM, preferably 0.1 to 200 mM. Solutions or fine powder of boron salts, boron based compounds or boron containing complexes can be used as a flame retardant, an antibacterial agent, an antiseptic, a sanitizer, an insect-repellent, an antiviral agent, or an organism-repellent and specific examples thereof include boric acid, borax, and copper borofluoride.

Besides these chemical agents, various sanitizers, insecticides or insect-repellents usually used can be used in this invention.

Specific examples of sanitizers or components include triazole derivatives such as azaconazole, ethaconazole, propiconazole, bromuconazole, difenoconazole, itraconazole, flutriaphor, myclobutanil, fenethanil, penconazole, tetraconazole, hexaconazole, tebuconazole, imibenccnazole, flusilazole, ribavirin, triamiphos, isazophos, triazophos, idinfos, fluotrimazole, triadimefone, triadimenol, diclobutrazol, diniconazole, diniconazole M, bitertanol, epoxiconazole, triticonazole, metconazole, ipconazole, furconazole, furconazole-cis, and cyproconazole, sulfonamides such as dichlorofluanid (euparene), tolyfluanid (methyl-euparen), cyclofluanide, folpet, and fluorofolpet, benzimidazoles such as carbendazim, benomyl, fuberidazole, thiabendazole, or salts thereof, thiocyanates such as thiocyanate methylthiobenzothiazole, and methylene bisthiocyanate, morpholine derivatives such as C11~C14-4-alkyl-2,6-dimethylmorpholine analogues (tridemorph), and (+/-)-cis-4-[3-(t-butylphenyl)-2-methylpropyl]-2,6-dimethylmorpholine (fenpropimorph, farimorph), organic iodine compounds such as 3-iodo-2-propyl-n-butylcarbamate, 3-iodo-2-propyl-n-hexylcarbamate, 3-iodo-2-propylcyclohexylcarbamate, 3-iodo-2-propylphenylcarbamate, p-chlorophenyl-3-iodopropargyl formal, 3-bromo-2,3-diiodo-2-propenylethylcarbonate (Sunplus), and 1-[(diiodomethyl)sulfonyl]-4-methylbenzene (amical), organic bromo derivatives such as bronopol, isothiazolines such as N-methylisothiazoline-3-one, 5-chloro-N-methylisothiazoline-3-one, 4,5-dichloro-N-octylisothiazoline-3-one, and N-octylisothiazoline-3-one (octhilinone), benzisothiazolines such as cyclopentaisothiazoline, pyridines such as 1-hydroxy-2-pyridinethione (or their sodium salts, iron salts, manganese salts, zinc salts, etc.), and tetrachloro-4-methylsulfonylpyridine, dialkyldithiocarbamates such as sodium or zinc salt of dialkyldithiocarbamate, and tetramethylthiuramdisulfide (TMTD), nitriles such as 2,4,5,6-tetrachloroisophthalonitrile (chlorothalonil), microbial agents having an activated halogen atom such as tectamer, bronopol, and brumidox, 2-mercaptobenzothiazoles, benzothiazoles such as dazomet, cyclodienes such as chlordane, dieldrin, aldrin, heptachlor, nitrosos such as N-nitroso-N-cyclohexylhydroxylamine, quinolines such as 8-hydroxyquinoline, benzyl alcohol mono(poly)hemiformal, oxazolidine, hexahydro-s-triazine, formaldehyde-generating substances such as N-methylolchloroacetamide, tris-N-(cyclohexyldiazenium dioxine)tributyl tin or potassium salt, bis-(N-cyclohexyl)diazinium-dioxine copper or aluminum, and the like.

Specific examples of insecticides agents or components, mothballs or their components which can be used in this invention include phosphoric acid esters such as azinphos-ethyl, azinphos-methyl, 1-(4-chlorophenyl)-4-(O-ethyl, S-propyl)phosphoryloxyprazole (TIA-230), chlorpyrifos, tetrachlorvinphos, coumaphos, dethomen-S-methyl, diazinon, dichlorvos, dimethoate, ethoprophos, etholimphos, fenitrothiun, pyridafenthion, heptenophos, parathion, parthion-methyl, propetamphos, phosalone, phoxim, pirimiphos-ethyl, pirimiphos-methyl, profenofos, prothiophos, sulprofos, triazofos, and trichlorfon, carbamates such as aldicarb, bendiocarb, 2-(1-methylpropyl)phenylmethylcarbamate, butocarboxim, butoxycarboxim, carbaryl, carbofuran, carbosulfan, chloethocarb, isoprocarb, methomyl, oxamyl, pyrimicarb, promecarb, propoxur, and thiodicarb, pyrethroids such as allethrin, alfamethrin, bioresmethrin, cycloprothrin, cyfluthrin, decamethrin, cyhalothrin, cypermethrin, deltamethrin, α-cyano-3-phenyl-2-methylbenzyl-2,2-dimethyl-2-(2-chloro-2-trifluoromethylvinyl)cyclopropane-1-propanecarboxylate, fenpropathrin, fenfluthrin, fenvalerate, flucythrinate, flumethrine, fluvalinate, permethrin, ethofenprox, and resmethrine, nitroiminos and nitromethylenes such as 1-(6-chloro-3-pyridinyl-methyl)-4,5-dihydro-N-nitro-1H-imidazol-2-amine (imidacloprid), and the like.

Also, insect hormones and IGR (insect growth regulator) and their derivatives can be used. These sanitizers, insecticides or insect-repellents may be used alone or in combination. The concentration of the sanitizers, insecticides or insect-repellents used in this invention in solutions upon treatment of porous articles is desirably adjusted depending on the intensity of physiological activity, the purpose of the treatment, and solubility of the chemical agent. Their concentration is usually 0.0001 to 20% by weight, preferably 0.001 to 5% by weight. While many of the chemical agents are volatile, it is possible to maintain potency of the chemical agents for a long period of time by imparting the chemical agents with or increasing slow-releasing properties, which is very useful.

As a fragrant, a deodorant, a rust preventive, an antibacterial agent, an antiseptic, a sanitizer, an insect-repellent, an antiviral agent, or an organism-repellent, can be used also extracts or extracted components from plants or synthetic substances having equivalent chemical agent structures to the extracted components of plants. Specific examples of such plants include trees such as a Japanese cypress, Aomori cypress, herbs, mustard, horse radish, bamboo, Iriomote thistle root, Yaeyama coconut root, and the like. These plants are treated by mill, pressing, boiling, steam distillation or the like process to obtain extracts or extracted components. Specific examples of the extracted components from plant or synthetic substances having equivalent chemical agent structures thereto include tropolones such as hinokitiol, monoterpenes such as α-pinene, β-pinene, camphor, menthol, limonene, borneol, α-terpinene, γ-terpinene, α-terpineol, terpinen-4-ol, and cineole, sesquiterpenes such as α-cadinol, and t-murol, polyphenols such as catechin, and tannin, naphthalene derivatives such as 2,3,5-trimethylnaphthalene, long chain aliphatic alcohols such as citronellol, aldehydes such as cinnamaldehyde, citral, and perillaldehyde, allyl compounds such as allyl isothiocyanate, and the like. Further, there can also be used pyroligneous acid obtained by roasting wood.

These extracts, extracted components from plants, and synthetic substances having chemical agent structures equivalent to the extracted components are very useful since they originally exist in plants so that when the porous article is an article derived from plants such as wood, processed woody material, chaffs, rush, straw, or bamboo, and when the raw material the enzyme having a polyphenol oxidizing activity acts on is a component derived from plants such as lignin or lignin derivatives, there can be obtained not only effect of sealing the pores in the porous article by the macromolecule but also desirable effect of resistance to elution or slow-releasing properties due to interaction between the extracts or extracted components from plants or synthetic substances having equivalent chemical agent structures to the extracted components and the porous article or raw materials. In particular, the treated porous articles produced from combinations of natural substances are highly safe to environment and human body and have high affinity for living organisms, so that they can be used for various applications and are very useful.

Further, as a rust preventive, an antibacterial agent, an antiseptic, a sanitizer, an insect-repellent, an antiviral agent, or an organism-repellent, there can be used aromatic compounds or cyclic compounds having one or more substituents selected from a hydroxyl group, an amino group, a halogen atom, and a nitro group. Based on the same principle as in the case of the extracts or extracted components from plants or synthetic substances having equivalent chemical agent structures to the extracted components, these aromatic compounds can have resistance to elution of chemical agents or chemical agent slow-releasing properties, which is useful in this invention.

Specific examples of the aromatic or cyclic compounds having one or more substituents selected from a hydroxyl group, an amino group, a halogen atom, and a nitro group include o-phenylphenol, 1-naphthol, 2-naphthol-o-chlorophenol, 2,4-dinitrophenol, 4,6-dinitro-o-cresol, pentachlorophenol, 2,3,5-trichlorophenol, 2,4,6-trichlorophenol, monochloronaphthalene, trichloronaphthalene, tetrachloronaphthalene, 2,4,5-trichlorophenyllaurate monochloronaphthalene, chloronitrophenol, chloronitrotoluene, o-dichlorobenzene, 1,3,5-trichlorobenzene, 1,2,4-trichlorobenzene, 2,4,6-tribromophenol, 4-bromo-2,5-dichlorophenol, bromo-o-phenylphenate, 4-chlorophenyl-3-iodopropargylformal, creosote oil, chlorinated terpene, butylhydroxyanisole, butylhydroxy-toluene, benzoic acid, p-hydroxybenzoic acid, methyl, ethyl, propyl, butyl, isobutyl, isopropyl or the like esters of p-hydroxybenzoic acid, and the like. Also, dehydroacetic acid and sorbic acid can be used in this invention as an antibacterial agent, an antiseptic, a sanitizer, an insect-repellent, an antiviral agent, or an organism-repellent.

Dimension stability, prevention of cracking, moisture conditioning properties, water absorption properties, water repellency, and surface smoothness are imparted or improved by controlling hydrophilic or hydrophobic properties of the macromolecule generated in the porous article in the treatment of porous article according to this invention. For example, macromolecules of lignosulfonic acid is macromolecurized into a hydrophilic high molecular gel in the porous article, the hydrophobic properties of which can be increased by using unsaturated compounds such as unsaturated fatty acids, unsaturated alcohols, unsaturated alkyl compounds, drying oils in combination as other components in the treatment agent. The hydrophobic properties can also be increased by using an aromatic compound having a saturated or unsaturated alkyl chain containing 1 to 22 carbon atoms as a substituent group in addition to a hydroxy group, more specifically urushiol, as a major component for macromolecularization by the polyphenol oxidizing enzyme or by adding such a compound to lignin or lignin derivatives.

Alternatively, a phenolic compound and/or aromatic amine compound having a substituent containing a moiety of polyoxyethylene or polyethyleneimine, in addition to a hydroxyl group and/or an amino group may be used as a major component for macromolecularization by a polyphenol oxidizing enzyme or added to lignin or lignin derivatives. This is very useful since water retention of the porous article after the treatment can be increased or in the case of treating wood, dimension stability and crack preventing properties can be imparted or increased. Particularly when the phenolic compound and/or aromatic amine compound such as lignin or lignin derivatives is used as a major component for macromolecularization by the polyphenol oxidizing enzyme, there can be used aromatic compounds having a substituent containing a moiety of polyoxyethylene, polyethyleneimine or saturated or unsaturated alkyl chain with 1 to 22 carbon atoms as an additive for modifying the physical properties of the macromolecule

The aromatic compounds having a substituent containing a moiety of polyoxyethylene or polyethyleneimine can be obtained by reacting an aromatic compound having a hydroxyl group, an amino group, a carboxyl group or the like as structural portion with ethylene oxide or ethyleneimine. Also, such aromatic compounds can be obtained by reacting an aromatic compound having an aldehyde group as a substituent on the aromatic ring or as a functional group on the substituent on the aromatic ring, such as vaniline, o-vaniline, 3,4-dihydroxybenzaldehyde, benzaldehyde, and 2-phenylpropionaldehyde with polyethyleneimine to form a Schiff's base.

Imparting or improvement of ion exchanging properties by the treatment of a porous article according to this invention can be achieved by controlling the anionic or cationic properties of the macromolecule generated in the porous article. For example, the macromolecule of lignosulfonic acid, which has a cation exchange ability, can improve the cation exchange ability of porous articles derived from plants, such as wood, processed woody material, chaffs, rush, straw, and bamboo. Further, imparting or improvement of anion exchange ability can be achieved by adding to lignin or lignin derivatives an aromatic compound having an amino group, such as o-phenylenediamine, p-phenylenediamine, 3,4-diaminobenzophenone, o-aminophenol, p-aminophenol, 1,2-diaminoanthraquinone, or 1,4-diaminoanthraquinone, or an aromatic compound having a substituent containing a moiety of quaternary ammonium salt or polyethyleneimine, or by macromolecularizing an aromatic amine compound by polyphenol oxidizing enzyme.

The treated porous article according to this invention has an ability to physically seal diffusion of folmaldehyde from the inside of the porous article or from other materials which the porous article contacts to open air. Also, the treatment for imparting or improving the anion exchange ability and allowing formaldehyde to react with the amino groups in the treatment agent, thereby imparting or improving formaldehyde absorption properties. Polyphenol substances such as catechin are known to react with methylmercaptane, trimethylamine, ammonia and malodorous substances such as that which is a source of tobacco smell, and deodorize them. Therefore, the polyphenol substances are useful in applications of this invention intended for deodorization. Such reactions with gaseous substances proceed more efficiently when the reaction site has a larger surface area and hence use of the method for treating a porous article according to this invention makes it possible to produce articles having high deodorizing effect.

As the chemical agents which can be used for imparting or improving flame retarding properties by the method for treating a porous article according to this invention, there can be used many of known flame retardants, for example, phosphates, hydrogen phosphates, sulfates, hydrogen sulfates, carbonates, borates, silicates, nitrates, fluorides, chlorides, bromides, and hydroxides which have a cationic moiety of, for example, Na, K, Mg, Ca, Ba, Al, Zn, Cu, Mn, Ni, Si, Sn, Pb, or the like element, and more specifically, aluminum hydroxide, magnesium hydroxide, zirconium hydroxide, antimony trioxide, barium metaborate, tin oxide, red phosphorus, ammonium phosphate. In particular, when lignosulfonic acid is used as the phenolic compound or aromatic amine compound, lginosulfonic acid contains fine particles of calcium carbonate, calcium hydroxide, magnesium carbonate or magnesium hydroxide since the boiling process in pulp plant is performed mainly using calcium sulfite or magnesium sulfite. The method for treating a porous article according to this invention enables efficient utilization of the fine particles as a flame retardant and therefore is very useful.

Utilizing the method for treating a porous article according to this invention, vessels can be manufactured from materials derived from natural substances having biodegradability, such as wood chips, wood powder, chaffs, rush, straw, bamboo, fibers, paper, pulp and the like. It is desirable to adjust the moisture controlling properties, water absorption properties, water repellency, surface smoothness, bioaffinity, or ion exchange ability of these vessels depending on the purpose. Since this invention uses enzymatic macromolecularization processes, it is highly safe to human body and the environment and the vessels manufactured can be used in various fields. They are useful particularly in such a field as requiring biodegradability in soil, compost or the like. When the vessels of this invention are used as pots for horticulture, breeding can be performed in the pots and the plants can be transplanted to the ground together with the pots since the pots are biodegraded so that work for transplantation can be saved.

Coloring by the method for treating a porous article according to this invention can be achieved by dyeing/coloring wood fixedly by reacting a dye or its precursor on which a polyphenol oxidizing enzyme can act, such as o-phenylenediamine, p-phenylenediamine, catechol, gallic acid, and quercetin, and the polyphenol oxidizing enzyme with wood to generate a coloring substance in the wood, or converting a coloring substance and a polyphenol compound such as lignin already contained in the wood to a composite macromolecule. In the above-described wood dyeing or coloring treatment, many polyphenol oxidizing enzymes are known to bleach lignin, which is a coloring substance contained in wood, hence, the wood dyeing/coloring treatment of this invention is very useful since it allows simultaneous practice of enzymatic bleaching and dyeing/coloring treatment so that the process can be shortened and color hue can be improved. Further, use of the enzyme having a polyphenol oxidizing activity and lignin or lignin derivatives such as lignosulfonic acid or lignosulfonate in the treatment of wood makes it possible to decrease a difference in the color hue or chromaticity between the heart wood portion and sap wood portion, thereby providing wood having a more uniform natural-feeling coloring.

The treatment agent for treating a porous article according to this invention is a composition containing an enzyme having a polyphenol oxidizing activity, a reaction substrate for the polyphenol oxidizing enzyme system, a phenolic compound and/or an aromatic amine compound, an unsaturated compound, a chemical agent, and the like as described-above, which may further contain a pH adjusting agent, a dye, a polymer, a solid or the like, if desired.

The treatment agent for treating a porous article according to this invention can be prepared as one pack agent by mixing the above-described composition as powder or granulated powder. Granulation is formulation conducted for suppressing dusting, for imparting the storage stability of the treatment agent, or for the sake of convenience for some purposes. More specifically, granulation can be carried out by any granulation operation such as marume granulation, extrusion granulation, flow granulation, centrifugal flow granulation or the like depending on the purpose. In this case, in order to increase the storage stability of the enzyme having a polyphenol oxidizing activity in the treatment agent, it is effective to granulate the enzyme separately from other components of the treatment agent but together with an enzyme stabilizer. The treatment agent for treating a porous article according to this invention can be prepared as a concentrated solution which is to be diluted upon use or a solution having an appropriate concentration which can be used without dilution. In this case, in order to prevent oxidation of the treatment agent before use, it is desirable to place the treatment agent in a vessel with sealing upon storage so that contact with open air can be avoided. Further, it is desirable to suppress oxidation upon the manufacture of the treatment agent by deaeration, for example. When the treatment agent for treating a porous article according to this invention is prepared, it is also possible to prepare the enzyme having a polyphenol oxidizing activity separately from the other components and mixing them immediately before use.

### BEST MODE FOR CARRYING OUT THE INVENTION

Hereafter, representative examples of this invention are described in greater detail. However, these examples are exemplary and this invention should not be construed as being limited thereto. In the following examples, all % are by weight unless otherwise indicated specifically.

### Example 1: Cultivation and Concentration

In a cultivation tank containing 3-liter medium consisting of 0.5% glucose, 0.1% NaNO₃, 1.34% Na₂HPO₄·12H₂O, 0.3% KH₂PO₄ 0.1% NaCl, 0.2% peptone, 20 ppm yeast extract, 0. 01% MgSO₄·7H₂O, and 0.1 mM CuSO₄, adjusted to pH 8 by addition of 10% NaOH, was inoculated *Myrothecium verrucaria* SD3001 (Accession No. FERM BP-5520) and cultivation was continued at 28 °C for 3 days with shaking. After the cultivation, the culture medium was centrifuged at 4 °C to obtain 2.5 liters of cell-free culture broth.

Then, an aliquot of the culture broth was concentrated by Minitan ultrafiltration system (Millipore Co.) using Minitan Filter Packet (CAT. NO.: PTGCOMPO4, Millipore Co.) as a fraction of a molecular weight of 10,000 or more. Further, this fraction was dialyzed against 200 ppm NH₄HCO₃ and then freeze-dried to obtain a crude purification product as a freeze-dried product. The freeze-dried product had a polyphenol oxidase activity of 10 U/mg.

### Example 2: Cultivation and Concentration

In a cultivation tank containing 3-liter medium consisting of 0.5% glucose, 0.1% NaNO₃, 1.34% Na₂HPO₄·12H₂O, 0.3% KH₂PO₄ 0.1% NaCl, 0.2% peptone, 20 ppm yeast extract, 0.01% MgSO₄·7H₂O, and 0.1 mM CuSO₄, adjusted to pH 8 by addition of 10% NaOH, was inoculated *Myrothecium roridum* SD3002 (Accession No. FERM BP-5523) and cultivation was continued at 28 °C for 3 days with shaking. After the cultivation, the culture medium was centrifuged at 4 °C to obtain 2.5 liters of cell-free culture broth.

Then, an aliquot of the culture broth was concentrated by Minitan ultrafiltration system (Millipore Co.) using Minitan Filter Packet (CAT. NO.: PTGCOMP04, Millipore Co.) as a fraction of a molecular weight of 10,000 or more.

Further, this fraction was dialyzed against 200 ppm NH₄HCO₃ and then freeze-dried to obtain a crude purification product as a freeze-dried product. The freeze-dried product had a polyphenol oxidase activity of 8 U/mg.

### Example 3: Treatment of Wood

A reaction mixture for treating wood containing 30 ppm of the freeze-dried product (10 U/mg) described in Example 1, 5% of commercially available lignosulfonic acid (LSA), 0.04 M copper (II) sulfate, and 0.08 M ethylenediamine (EDA) was prepared, to which were dipped Japan cedar blocks (3 cm x 3 cm x 2 cm, butt end: 3 cm x 3 cm) under reduced pressure for impregnation treatment. The pH was adjusted with sodium hydroxide or sulfuric acid.

The reduced pressure impregnation operation was performed by a handy method, i.e., by applying a reduced pressure of 650 to 700 mmHg for 1 hour after dipping the Japan cedar blocks in the treatment liquid and then keeping the blocks at atmospheric pressure for 30 minutes as they were dipped in the treatment liquid. By measuring the weights of each block before and after the impregnation treatment, it was confirmed that as a result of the pressure reduction treatment operation, a sufficient amount of the treating liquid (10 to 14 g) was injected in the blocks.

Further, the wood blocks after the impregnation treatment were placed in an incubator at 28 °C, for 5 days to effect drying and macromolecularization reaction. Thereafter, 200 ml of water was added to each wood block and water was stirred at 25 ± 3 °C for 8 hours using a magnetic stirrer in such a state that the blocks were submerged under water surface in order to effect a leaching operation. After the leaching operation, the water was measured of absorbance at 280 nm to record the amount of lignosulfonic acid which leached off and the amount of the chemical agent which leached off was determined by complex formation with PAN (1-(2-pyridylazo),-2-naphthol, Aldrich Chemical Company) and spectrophotometry. Results are shown in Table 1. For comparison, results with the treatment agent containing no polyphenol oxidizing enzyme are shown in Table 1.

It was revealed that wood treatment under reduced pressure using a polyphenol oxidizing enzyme and lignosulfonic acid enables efficient injection and fixation of chemical agents and these effects are greater on an alkali pH side not lower than pH 8.

**Table 1**

| Reaction Mixture Composition for Wood Treatment | | Enzyme | pH of Treating Agent | Elution Rate(%) | |
|---|---|---|---|---|---|
| LSA | Chemical Agent Used And Its Concentration | | | LSA | Copper |
| 5% | 0.04M CuSO₄ + 0.08M EDA | added | 7.0 | 38.2 | 42.1 |
| 5% | 0.04M CuSO₄ + 0.08M EDA | not added | 7.0 | 43.6 | 52.1 |
| 5% | 0.04M CuSO₄ + 0.08M EDA | added | 7.5 | 12.6 | 12.2 |
| 5% | 0.04M CuSO₄ + 0.08M EDA | not added | 7.5 | 38.3 | 41.5 |
| 5% | 0.04M CuSO₄ + 0.08M EDA | added | 8.2 | 0.7 | 3.5 |
| 5% | 0.04M CuSO₄ + 0.08M EDA | not added | 8.2 | 13.2 | 29.8 |
| 5% | - | added | 8.5 | 0.4 | - |
| 5% | - | not added | 8.5 | 55.2 | - |
| - | 0.04M CuSO₄ | not added | 4.1 | - | 58.5 |

### Example 4: Treatment of Wood

A reaction mixture for treating wood containing 30 ppm of the freeze-dried product (10 U/mg) described in Example 1, 5% of commercially available lignosulfonic acid (LSA) , and one or more of various chemical agents was prepared. Japan cedar wood blocks were subjected to reduced pressure impregnation treatment with the reaction mixture thus obtained, drying and macromolecularization reaction, and leaching treatment in the same manner as in example 3. For chemical agents having low solubilities, they were added to aqueous lignosulfonic acid solution after it was warmed to 60 to 90 °C, and it was cooled at 25 °C after the chemical agents were suspended, dispersed, dissolved by Vortex-Mixer, and the polyphenoloxydase freeze-dried product was added to obtain treatment solution.

The amount of lignosulfonic acid which leached off was determined by measurement of the absorbance at 280 nm, and the amount of the chemical agent which leached off was determined by complex formation and spectrophotometry using PAN (1-(2-pyridylazo)-2-naphthol, Aldrich Chemical Company) or quinalizarin (Wako Pure Chemicals), separation, identification and determination, using HPLC or gas chromatography, or atomic absorption analysis were performed, followed by calculation of the amount of the chemical agents which was eluted. When the chemical agents had absorption at 280 nm, the concentration of lignosulfonic acid was calculated by measuring the absorbance at 280 nm of the chemical agents of which the concentration was measured by the above-described method and subtracting an influence by the chemical agent from the absorbance at 280 nm of the liquid which was eluted. The amounts of the chemical agents and lignosulfonic acid which were eluted were calculated and compared taking the amounts of them injected upon the impregnation treatment as 100%. Results are shown in Table 2.

The results show that wood pressure treatment using a polyphenol oxidizing enzyme and lignosulfonic acid enables efficient injection and fixation of chemical agents and these effects are greater on an alkali pH side not lower than pH 8. Further, results obtained without polyphenol oxidizing enzyme (comparison) are shown in Table 3.

**Table 2 Elution Rate in the Presence of Enzyme**

| Chemical Agent Used And Its Concentration | pH of Treating Agent | Elution Rate (%) | |
|---|---|---|---|
| | | LSA | Chemical Agent |
| 0.04M CuSO₄ + 0.01M EDTA* | 8.5 | 5.9 | 10.4 (Copper) |
| 0.04M CuSO₄ + 0.01M EDTA* | 7.5 | 12.2 | 19.7 (Copper) |
| 0.04M CuSO₄ + 0.01M EDTA* | 7.0 | 20.3 | 28.8 (Copper) |
| 0.04M CuSO₄ + 0.01M Nitrilotriacetic acid | 8.2 | 4.3 | 5.5 (Copper) |
| 0.04M Copper carbonate + 0.08M EDA | 8.2 | 0.6 | 2.6 (Copper) |
| 0.04M ZnSO₄ + 0.08M EDA | 8.2 | 0.9 | 3.1 (Zinc) |
| 0.04M NiCl₂ + 0.08M EDA | 8.2 | 1.1 | 3.8 (Nickel) |
| 1% Boric acid | 8.6 | 0.7 | 25.1 (Boron) |
| 1000 ppm Hinokitiol | 8.5 | 0.6 | 3.1 |
| 1000 ppm Hinokitiol +0.01M CuSO₄ | 8.1 | 0.7 | 3.3 (H**) |
| 1000 ppm Hinokitiol +0.01M Ag₂SO₄ | 8.5 | 0.8 | 3.0 (H**) |
| 500 ppm (1R)-(+)-α-pinene | 8.5 | 0.6 | 0.3 |
| 500 ppm -α-terpineol | 8.5 | 0.7 | 0.6 |
| 500 ppm (1R,2S,5R)-(-)-menthol | 8.5 | 0.5 | 0.2 |
| 500 ppm cineole | 8.5 | 0.5 | 0.2 |
| 1000 ppm (+)-Catechin·H₂O | 8.8 | 2.7 | 2.8 |
| 500 ppm Tannic acid | 8.7 | 3.8 | 4.0 |
| 10 ppm 2,3,5-Trimethylnaphthalene | 8.5 | 0.8 | 0.1 |
| 100 ppm β-citronellol | 8.5 | 0.7 | 0.4 |
| 100 ppm citral | 8.5 | 2.5 | 1.8 |
| 100 ppm Cinnamaldehyde | 8.5 | 1.5 | 0.7 |
| 100 ppm Allyl isothiocyanate | 8.5 | 3.5 | 2.2 |
| 200 ppm o-Phenylenediamine | 8.5 | 1.0 | 0.2 |
| 200 ppm 1,3,5-Trichlorobenzene + T*** | 8.5 | 2.3 | 0.2 |
| 100 ppm 2,4,6-Tribromophenol + T*** | 8.5 | 2.2 | 0.1 |

| | | | |
|---|---|---|---|
| * EDTA; Ethylenediaminetetraacetic acid | | | |
| ** H; Hinokitiol | | | |
| *** T; Tween 80 (500 ppm) | | | |

**Table 3 Elution Rate in the Absence of Enzyme (Comparison)**

| Chemical Agent Used And Its Concentration | pH of Treating Agent | Elution Rate (%) | |
|---|---|---|---|
| | | LSA | Chemical Agent |
| 0.04M CuSO₄ + 0.01M EDTA* | 8.5 | 26.5 | 30.1 (Copper) |
| 0.04M CuSO₄ + 0.01M Nitrilotriacetic acid | 8.2 8.2 | 24.1 24.1 | 30.3 (Copper) |
| 0.04M Copper carbonate + 0.08M EDA | 8.2 | 12.0 | 25.6 (Copper) |
| 0.04M ZnSO₄ + 0.08M EDA | 8.2 | 15.3 | 14.9 (Zinc) |
| 0.04M NiCl₂ + 0.08M EDA | 8.2 | 16.6 | 17.2 (Nickel) |
| 1% Boric acid | 8.6 | 22.1 | 32.3 (Boron) |
| 1000 ppm Hinokitiol | 8.5 | 24.2 | 14.4 |
| 1000 ppm Hinokitiol +0.01M CuSO₄ | 8.1 | 23.0 | 19.5 (H**) |
| 1000 ppm Hinokitiol +0.01M Ag₂SO₄ | 8.5 | 19.0 | 17.8 (H**) |
| 500 ppm (1R)-(+)-α-pinene | 8.5 | 22.8 | 7.3 |
| 500 ppm -α-terpineol | 8.5 | 24.5 | 9.1 |
| 500 ppm (1R,25,5R)-(-)-menthol | 8.5 | 27.4 | 7.5 |
| 500 ppm cineole | 8.5 | 25.7 | 6.2 |
| 1000 ppm (+)-Catechin·H₂O | 8.8 | 23.4 | 11.3 |
| 500 ppm Tannic acid | 8.7 | 24.2 | 21.0 |
| 10 ppm 2,3,5-Trimethylnaphthalene | 8.5 | 23.3 | 5.6 |
| 100 ppm β-citronellol | 8.5 | 25.4 | 6.8 |
| 100 ppm citral | 8.5 | 23.4 | 7.1 |
| 100 ppm Cinnamaldehyde | 8.5 | 22.1 | 8.5 |
| 100 ppm Allyl isothiocyanate | 8.5 | 26.3 | 7.5 |
| 200 ppm o-Phenylenediamine | 8.5 | 16.5 | 5.3 |
| 200 ppm 1,3,5-Trichlorobenzene + T*** | 8.5 | 21.6 | 5.4 |
| 100 ppm 2,4,6-Tribromophenol + T*** | 8.5 | 20.3 | 5.1 |

| | | | |
|---|---|---|---|
| * EDTA; Ethylenediaminetetraacetic acid | | | |
| ** H; Hinokitiol | | | |
| *** T; Tween 80 (500 ppm) | | | |

(1R)-(+)-α-pinene, (1R,2S,5R)-(-)-menthol, cineole, (+)-catechin·H₂O, tannic acid, 2,3,5-trimethylnaphthalene, β-citronellol, citral, cinnamaldehyde, and allyl isothiocyanate were obtained from Aldrich Chemical Company, copper carbonate (copper (II) carbonate, basic), copper sulfate, zinc sulfate, nickel (II) chloride, boric acid, and silver sulfate were obtained from Wako Pure Chemicals Co., Ltd., hinokitiol, 1,3,5-trichlorobenzene, and 2,4,6-tribromophenol were obtained from Tokyo Kasei Kogyo Co., Ltd.

### Example 5: Treatment of Wood

A reaction mixture for treating wood containing 40 ppm of the freeze-dried product (8 U/mg) described in Example 2, 5% of commercially available lignosulfonic acid (LSA), and one or more of various chemical agents was prepared. Cypress wood blocks were subjected to reduced pressure impregnation treatment with the reaction mixture thus obtained, drying and macromolecularization reaction, and leaching treatment in the same manner as in example 3 and 4. The amounts of the chemical agents and lignosulfonic acid which were eluted were calculated and compared taking the amounts of them injected upon the impregnation treatment as 100%. Results are shown in Table 4.

The results show that injection and fixation of chemical agents as efficient as in Examples 3 and 4 can be performed and these effects are greater on an alkali pH side not lower than pH 8.

**Table 4**

| Chemical Agent Used And Its Concentration | pH of Treating Agent | Elution Rate (%) | |
|---|---|---|---|
| | | LSA | Chemical Agent |
| 0.04M CuSO₄ + 0.01M EDTA | 8.2 | 3.9 | 6.4 (Copper) |
| 0.04M CuSO₄ + 0.01M EDTA | 7.5 | 23.3 | 21.1 (Copper) |
| 1000 ppm Hinokitiol | 8.5 | 2.2 | 7.2 |
| 1000 ppm Hinokitiol +0.01M CuSO₄ | 8.2 | 2.1 | 5.6 (H) |
| 1000 ppm Hinokitiol +0.01M Ag₂SO₄ | 8.5 | 2.6 | 5.8 (H) |
| 1000 ppm (+)-Catechin·H₂O | 8.8 | 5.2 | 6.6 |
| 500 ppm Tannic acid | 8.7 | 6.8 | 8.7 |

### Example 6: Treatment of Wood

Cypress wood blocks were subjected to reduced pressure impregnating treatment using commercially available bilirubin oxidase (freeze-dried) (Sigma) as the polyphenol oxidizing enzyme, drying/macromolecularization, and leaching treatment in the same manner as in Examples 3 and 4. Taking the amounts of the lignosulfonic acid (LSA) and chemical agent as injected upon the impregnation treatment as 100%, respectively, the amounts of the substances which were eluted were compared. The results are shown in Table 5.

The results show that the injection and fixation of the chemical agents as effective as in Examples 3 and 4 are possible and that the effects are greater on the alkaline pH region of pH 8 or higher.

**Table 5**

| Chemical Agent Used And Its Concentration | pH of Treating Agent | Elution Rate (%) | |
|---|---|---|---|
| | | LSA | Chemical Agent |
| 0.04M CuSO₄ + 0.01M EDTA | 8.5 | 7.2 | 12.5 (Copper) |
| 0.04M CuSO₄ + 0.01M EDTA | 7.5 | 15.4 | 23.5 (Copper) |
| 0.04M CuSO₄ + 0.01M EDTA | 7.0 | 25.1 | 29.5 (Copper) |
| 0.04M CuSO₄ + 0.01M EDA | 8.1 | 2.0 | 5.3 (Copper) |
| 0.04M CuSO₄ + 0.01M EDA | 7.5 | 12.5 | 13.8 (Copper) |
| 0.04M CuSO₄ + 0.01 M Nitrolotriace acid | 8.2 | 6.8 | 5.7 (Copper) |
| 0.04M Copper carbonate + 0.08M EDA | 8.2 | 1.6 | 4.1 (Copper) |
| 0.04M ZnSO₄ + 0.08M EDA | 8.2 | 1.2 | 3.3 (Zinc) |
| 0.04M NiCl₂ + 0.08M EDA | 8.2 | 1.4 | 4.1 (Nickel) |
| 1% Boric acid | 8.6 | 3.7 | 26.2 (Boron) |
| 1000 ppm Hinokitiol | 8.5 | 1.7 | 5.4 |
| 1000 ppm Hinokitiol +0.01M CuSO₄ | 8.1 | 1.9 | 5.5 (H) |
| 1000 ppm Hinokitiol +0.01M Ag₂SO₄ | 8.5 | 2.4 | 5.4 (H) |
| 500 ppm (1R) - (+) -α-pinene | 8.5 | 1.0 | 1.2 |
| 500 ppm α-terpineol | 8.5 | 1.1 | 1.4 |
| 500 ppm (1R,2S,5R)-(-)-menthol | 8.5 | 2.8 | 1.3 |
| 500 ppm cineole | 8.5 | 0.8 | 0.6 |
| 1000 ppm (+)-Catechin·H₂O | 8.8 | 4.4 | 6.3 |
| 500 ppm Tannic acid | 8.7 | 6.6 | 8.3 |
| 10 ppm 2,3,5-Trimethylnaphthalene | 8.5 | 2.4 | 0.3 |
| 100 ppm β-citronellol | 8.5 | 2.9 | 0.8 |
| 100 ppm citral | 8.5 | 7.9 | 6.0 |
| 100 ppm Cinnamaldehyde | 8.5 | 5.4 | 2.1 |
| 100 ppm Allyl isothiocyanate | 8.5 | 8.5 | 7.8 |
| 200 ppm o-Phenylenediamine | 8.5 | 2.5 | 0.6 |
| 200 ppm 1,3,5-Trichlorobenzene + T | 8.5 | 3.5 | 0.3 |
| 100 ppm 2,4,6-Tribromophenol + T | 8.5 | 3.3 | 0.3 |

### Example 7: Treatment of Wood

Cypress wood blocks were subjected to reduced pressure impregnating treatment using 5 U/ml peroxidase and 5 U/ml of alcohol oxidase as the polyphenol oxidizing enzyme system and 1% methanol, drying/macromolecularization, and leaching treatment in the same manner as in Examples 3 and 4. Taking the amounts of the lignosulfonic acid (LSA) and chemical agent as injected upon the impregnation treatment as 100%, respectively, the amounts of the substances which were eluted were compared. The results are shown in Table 6. Table 6 shows that the effective injection and fixation of the chemical agents are possible.

The peroxidase used was obtained from horse radish (Type II, Sigma) and the alcohol oxidase used was obtained from *Candida boidini* (Boehringer Mannheim Biochemica).

**Table 6**

| Chemical Agent Used And Its Concentration | PH of Treating Agent | Elution Rate (%) | |
|---|---|---|---|
| | | LSA | Chemical Agent |
| 0.04M CuSO₄ + 0.01M EDTA | 8.5 | 10.9 | 15.2 (Copper) |
| 0.04M CuSO₄ + 0.01M EDA | 8.1 | 5.2 | 10.3 (Copper) |
| 0.04M CuSO₄ + 0.01M Nitritoltriacetic acid | 8.2 | 10.1 | 7.7 (Copper) |
| 0.04M Copper carbonate + 0.08M EDA | 8.2 | 3.8 | 5.0 (Copper) |
| 0.04M ZnSO₄ + 0.08M EDA | 8.2 | 4.4 | 8.1 (Zinc) |
| 1% Boric acid | 8.6 | 5.6 | 27.5 (Boron) |
| 1000 ppm Hinokitiol | 8.5 | 4.5 | 6.2 (H) |
| 1000 ppm (+)-Catechin·H₂O | 8.8 | 5.3 | 8.7 |
| 500 ppm Tannic acid | 8.7 | 9.2 | 10.3 |

### Example 8: Treatment of Wood

A reaction mixture for treating wood containing 30 ppm of the freeze-dried product described in Example 1, 5% of commercially available lignosulfonic acid (LSA), and 2,000 ppm of tung oil was prepared, and Japan cedar wood blocks were subjected to reduced pressure impregnation treatment with the reaction mixture thus obtained, drying and macromolecularization reaction, and leaching treatment in the same manner as in examples 3 and 4. The amounts of the chemical agents and lignosulfonic acid which were eluted were calculated and compared taking the amounts of them injected upon the impregnation treatment as 100%. Results are shown in Table 7, which demonstrates a further improvement in fixation of the chemical agents.

**Table 7**

| Chemical Agent Used And Its Concentration | pH of Treating Agent | Elution Rate (%) | |
|---|---|---|---|
| | | LSA | Chemical Agent |
| 0.04M CuSO₄ + 0.08M EDA | 8.1 | 1.5 | 4.4 (Copper) |
| 0.04M ZnSO₄ + 0.08M EDA | 8.2 | 1.0 | 3.0 (Zinc) |
| 0.04M NiCl₂ + 0.08M EDA | 8.2 | 1.0 | 3.5 (Nickel) |
| 1000 ppm Hinokitiol | 8.5 | 1.1 | 0.5 |
| 1000 ppm Hinokitiol +0.01M CuSO₄ | 8.1 | 1.0 | 0.6 (H) |
| 500 ppm (1R)-(+)-α-pinene | 8.5 | 0.7 | 0.2 |
| 500 ppm α-terpineol | 8.5 | 0.6 | 0.3 |
| 500 ppm (1R,2S,5R)-(-)-menthol | 8.5 | 0.6 | 0.4 |
| 500 ppm cineole | 8.5 | 0.5 | 0.2 |
| 100 ppm β-citronellol | 8.5 | 1.7 | 0.2 |
| 100 ppm Cinnamaldehyde | 8.5 | 1.9 | 0.2 |
| 100 ppm Allyl isothiocyanate | 8.5 | 2.1 | 1.3 |
| 200 ppm 1,3,5-Trichlorobenzene + T | 8.5 | 1.2 | 0.2 |

### Example 9: Treatment of Wood

To 10 g of horse-radish root crushed by a mixer was added 10 ml of water and the mixture was filtered through a cloth to obtain a horse-radish extract solution. A reaction mixture for treating wood was prepared which contained 30 ppm of the freeze-dried product described in Example 1, 5% of commercially available lignosulfonic acid (LSA), and 5% of the above-described horse-radish extract solution, and Japan cedar wood blocks were subjected to reduced pressure impregnation treatment with the reaction mixture thus obtained, drying and macromolecularization reaction, and leaching treatment in the same manner as in example 3. The amount of the lignosulfonic acid which was eluted was calculated taking the amount of it injected upon the impregnation treatment as 100%. It was shown that the amount of lignosulfonic acid which was eluted remained 3.2% as a result of progress of macromolecularization reaction inside the wood and there was obtained treated wood product which contained horse-radish extract.

### Example 10: Treatment of Wood

A reaction mixture for treating wood containing 30 ppm of the freeze-dried product described in Example 1, 5% of commercially available lignosulfonic acid, and one or more various chemical agents was prepared, and Japan cedar wood blocks (3 cm x 3 cm x 2 cm, butt end: 3 cm x 3 cm) were subjected to pressure reduction (initial vacuum) and impregnation treatment under pressure using the reaction mixture in the same manner as in Examples 3 and 4.

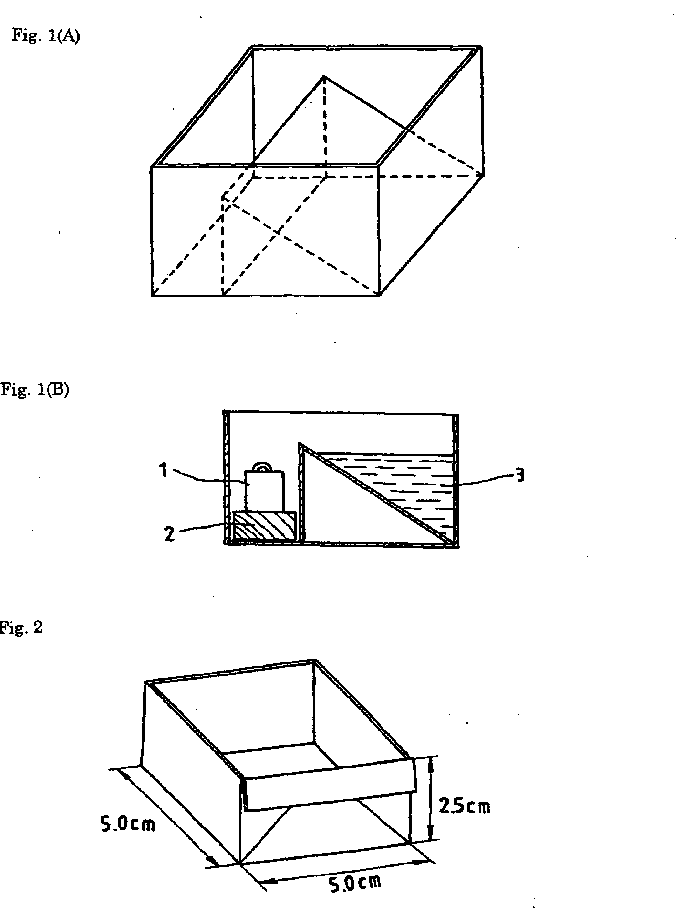

The pressure reduction and pressurization were performed by setting a vessel for impregnation operation as shown in Fig. 1 in a pressure reactor (5 liter in volume), conducting initial vacuum from 600 to 720 mmHg for 30 minutes using a vacuum pump, tilting the whole apparatus to allow the treatment liquid to flow toward the wood so that the wood blocks can be dipped in the treatment liquid, and then pressurized at 10 atm for 1 hour by flowing high pressure nitrogen gas in the reactor.

The wood blocks after the impregnation treatment were further subjected to drying/macromolecularization reaction and leaching treatment in the same manner as in Examples 3 and 4. The amounts of the chemical agents and lignosulfonic acid which were eluted were calculated and compared taking the amounts of them injected upon the impregnation treatment as 100%. Results are shown in Table 8, which shows that effective injection and fixation of the chemical agents are possible.

**Table 8**

| Chemical Agent Used And Its Concentration | Enzyme | pH of Treating Agent | Elution Rate (%) | |
|---|---|---|---|---|
| | | | LSA | Chemical Agent |
| - | not added | 8.5 | 26.3 | - |
| - | added | 8.5 | 0.3 | - |
| 0.04M CuSO₄ + 0.08M EDA | added | 8.1 | 0.6 | 3.0 (Copper) |
| 0.04M ZnSO₄ + 0.08M EDA | added | 8.2 | 0.8 | 2.8 (Zinc) |
| 0.04M NiCl₂ + 0.08M EDA | added | 8.2 | 0.9 | 3.2 (Nickel) |
| 1000ppm Hinokitiol | added | 8.5 | 0.4 | 2.3 |
| 1000ppm Hinokitiol CuSO₄ +0.01M CUSO₄ | added | 8.1 1 | 0.4 4 | 2.5 (H) |
| 1000ppm (+)-Catechin·H₂O | added | 8.8 | 2.1 | 2.0 |
| 500 ppm Tannic acid | added | 8.7 | 2.3 | 3.1 |

### Example 11: Antifungal Test On Treated Wood

In a 500 ml glass beaker, an incubator, charged with 100 ml of agar medium (pH 6.5) containing 4% glucose, 1.5% malt extracts, 0.3% peptone, and 2% agar was inoculated *Tyromyces palustris* FEPRI 0507 or *Coriolus versicolor* FEPRI 1030 (both obtained from Forestry and Forest Products Research Institute, Ministry of Agriculture, Forestry and Fisheries) and cultivation was continued at 26 °C for 1 week. The wood blocks after the leaching treatment obtained in Example 10 were placed on the cultured fungus with the direction of the fiber vertical, directly on the fungus in the case of the *Coriolus versicolor* strain or through an about 1 mm thick sterilized heat-resistant plastic net placed on the fungus in the case of the *Tyromyces palustris* strain, and then incubated at 26 °C for 12 weeks to effect antifungal treatment. Before the antifungal operation, the wood blocks after the leaching obtained in Example 10 were placed in a drier at 60 °C for 48 hours and then in a desiccator for 30 minutes to dry sufficiently and the weight of the wood blocks before the antifungal operation was measured. After completion of the antifungal operation, the wood blocks were taken out of the incubator and hyphae on the surface thereof were removed sufficiently. After air dried for about 24 hours, the wood blocks were dried sufficiently using a drier and a desiccator in the same manner as described above and their weight was measured. Percent weight loss (%) was calculated by comparing the weight obtained with the weight before the antifungal operation. The results are shown in Table 9, which shows that the method for treating wood according to this invention can impart antifungal properties to wood.

**Table 9**

| Conditions for Treating Wood | | | | Weight Loss(%) | |
|---|---|---|---|---|---|
| LSA | Chemical Agent Used And Its Concentration | Enzyme | pH of Treating Liquid | *Tyromyces palustris* | *Coriolus versicolor* |
| added | - | not added | 8.5 | 38.8 | 30.5 |
| added | - | added | 8.5 | 25.1 | 22.4 |
| added | 0.04M CuSO₄ + 0.08M EDA | added | 8.1 | 3.9 | 1.1 |
| added | 0.04M ZnSO₄ + 0.08M EDA | added | 8.2 | 5.3 | 3.1 |
| added | 0.04M NiCl₂ + 0.08M EDA | added | 8.2 | 1.6 | 0.7 |
| added | 1000ppm Hinokitiol | added | 8.5 | 2.2 | 1.5 |
| added | 1000ppm Hinokitiol + 0.01M CuSO₄ | added | 8.1 | 0.8 | 0.4 |
| added | 1000 ppm (+)-Catechin·H₂O | added | 8.8 | 9.9 | 8.3 |
| added | 500 ppm Tannic acid | added | 8.7 | 12.9 | 10.8 |
| Nontreatment | | | | 45.3 | 33.5 |

### Example 12: Treatment of Wood

A reaction mixture for treating wood containing 30 ppm of the freeze-dried product described in Example 1 and 2% of commercially available lignosulfonic acid (LSA) was prepared, and Japan cedar wood blocks were subjected to reduced pressure and pressure impregnation treatment with the reaction mixture thus obtained, drying and macromolecularization reaction in the same manner as in example 10. The reaction mixture was adjusted to pH 8.5. Then, an aqueous solution containing 0.4% (W/V) polyethyleneimine (Aldrich Chemical Company, average molecular weight: 700) and 0.02 M copper sulfate was prepared, which was used in the same reduced pressure and pressure treatment as in Example 10 to perform the second stage impregnation operation. After air-drying the wood blocks thus treated for 6 days, they were subjected to the same leaching treatment as in Example 3. The amount of copper ion which was eluted was calculated taking the amount of the copper ion injected upon the impregnation treatment as 100%. As a result, the amount of copper ion which was eluted remained to be 1.2%. This indicates that the macromolecularization and fixation of lignosulfonic acid inside the wood and formation of complexes from lignosulfonic acid, copper ion, and polyethyleneimine causes the copper ions to fix to the inside of the wood firmly.

Further, as the first stage impregnation operation, an aqueous solution containing 5% (W/V) polyethyleneimine and 0.25 M copper sulfate was injected to Japan cedar wood blocks in the same manner as in Example 9 and the wood blocks were air-dried for 6 days, followed by coating a reaction mixture containing 30 ppm of the freeze-dried product described in Example 2 and 10% of commercially available lignosulfonic acid on the wood blocks and allowing to stand in an incubator at constant temperature and constant humidity of 28 °C, 80% relative humidity for 3 days so that enzymatic macromolecularization reaction could proceed. As a result, the wood blocks thus obtained were prevented of migration of copper complex to the surface of the wood blocks and given a more natural brown surface color.

### Example 13: Treatment of Wood

A reaction mixture for treating wood containing 30 ppm of the freeze-dried product described in Example 1, 5% of commercially available lignosulfonic acid (LSA) , and 0.5% of p-phenylenediamine dihydrochloride (Kanto Kagaku Co., Ltd.) was prepared and Japan cedar wood blocks (3 cm x 3 cm x 10 cm, butt end: 3 cm x 3 cm) having a heart wood portion in part were subjected to reduced pressure and pressure treatment with the reaction mixture thus obtained in the same manner as in Example 10, and further to drying and macromolecularization reaction and leaching treatment in the same manner as in example 3. As a result, impregnation, coloring and fixation occurred more strongly in the sapwood portion of the wood and, hence, differences in color hue and chromaticity between the heart wood and sapwood became small so that wood having a more uniform, brown-colored one was obtained.

### Example 14: Treatment of Wood

An aqueous solution of commercially available lignosulfonic acid adjusted to 40% (W/V) was centrifuged (at 8000 g, for 15 minutes), filtered through Kiriyama Funnel Filter Paper No. 4 (Kiriyama Seisakusho, Ltd.), or filtered using a hollow fiber cartridge (Amicon, Inc., Type H5MP01-43 or H5MP100-43 (having a fractionating ability at 0.1 µm or MW 100, 000 each)) to obtain a supernatant or filtrate. Using this, there was prepared a reaction mixture for treating wood (pH 8.5) containing 5% of lignosulfonic acid and 30 ppm of the freeze-dried product described in Example 1, and Japan cedar wood blocks (3 cm x 1 cm x 4 cm, butt end: 3 cm x 1 cm) with the butt end surfaces sealed with commercially available epoxy adhesive were subjected to the same reduced pressure and pressure treatment as in Example 10. The weight of the injected treatment liquid was calculated from the change in weight before and after the impregnation treatment. As a result, there was observed an increase in the amount of the injected treatment liquid due to centrifugation, filtration or ultrafiltration. The results are shown in Table 10.

The treated wood blocks were further subjected to drying/macromolecularization reaction and leaching treatment in the same manner as in Example 3. The amount of the lignosulfonic acid which was eluted was obtained and compared taking the amounts of it injected upon the impregnation treatment as 100%. All the wood blocks showed low values of leaching rate of 0.2 to 0.5%.

**Table 10**

| Treatment Method | Amount of Injected Liquid |
|---|---|
| Centrifugation | 6.7 g |
| Filtration with filter paper | 6.4 g |
| Filtration with hollow fiber cartridge (H5MP01-43) | 7.2 g |
| Filtration with hollow fiber cartridge (H5MP100-43) | 8.3 g |
| No treatment (Control) | 5.1 g |

### Example 15: Treatment of Wood

A reaction mixture (pH8.5) for treating wood containing 30 ppm of the freeze-dried product described in Example 1, 5% of commercially available lignosulfonic acid (LSA), and 5% (W/V) of aluminum hydroxide powder having a particle diameter less than 0.1 µm was prepared and Japan cedar wood blocks whose butt end surfaces were sealed were subjected to reduced pressure and pressure treatment with the reaction mixture thus obtained, and drying and macromolecularization reaction in the same manner as in Example 14. The wood blocks thus obtained or non-treated wood blocks were placed in the flame of a gas burner for 10 seconds and conditions of surface of the wood blocks were observed. As a result, the carbonized portions of the treated wood blocks were clearly smaller than the carbonized portions of non-treated wood blocks, which demonstrates that the treated wood blocks were given flame retarding properties.

### Example 16: Treatment of Wood

A reaction mixture (pH 8.5) for treating wood was prepared from lignin (alkali) or lignin (organosorv)(both available from Aldrich Chemical Company) as lignosulfonic acid and 30 ppm of the freeze-dried product described in Example 1, and Japan cedar wood blocks were subjected to reduced pressure impregnation treatment with the reaction mixture thus obtained, drying and macromolecularization reaction, and leaching treatment in the same manner as in example 3. The amounts of the lignosulfonic acid which were eluted were calculated and compared taking the amounts of it injected upon the impregnation treatment as 100%. The results are shown in Table 11. In the case of lignosulfonic acid derivatives, fixation effects by enzyme are observed as in the case of lignosulfonic acid.

**Table 11**

| Lignosulfonic Acid (Derivative) Used | Enzyme | Elution Rate of LSA(%) |
|---|---|---|
| 5% Lignin (Alkali) | added | 0.3 |
| 2% Lignin (Alkali)+ 3% Lignin(Organosorb) | added | 3.6 |
| 5% Lignin (Alkali) | not added | 24.5 |
| 2% Lignin (Alkali)+ 3% Lignin (Organosorb) | not added | 22.3 |

### Example 17: Treatment of Wood

5.0 g of o-vaniline and 11.48 g of polyethyleneimine (average molecular weight: 700) (both available from Aldrich Chemical Company) were mixed and 20 ml of deionized water was added slowly to the mixture with stirring. The mixture was heated at 90 °C for 24 hours to form Schiff's base. A reaction mixture (pH 8.7) for treating wood containing 1% (W/V) of the reaction mixture thus obtained and 30 ppm of the freeze-dried product described in Example 1 was prepared and Japan cedar wood blocks were subjected to reduced pressure impregnation treatment, drying/macromolecularization reaction, and leaching treatment. The amount of the o-vanillin polyethyleneimine derivative which was eluted was calculated and compared taking the amounts of it injected upon the impregnation treatment as 100%. The amount of o-vanillin polyethyleneimine derivative which was eluted was 3.5%. On the other hand, the leaching amount was 19.8% for the wood blocks treated with the treatment liquid containing no polyphenol oxidizing enzyme. Thus, fixation effects were observed because of the progress of macromolecularization reaction inside the wood.

### Example 18: Rice Straw Treated

A 50 ml reaction mixture (pH 8.1) containing 30 ppm of the freeze-dried product described in Example 1, 15% of commercially available lignosulfonic acid (LSA), 40 mM of copper sulfate, and 40 mM of ethylenediamine was prepared. Then 5 g of sufficiently dried rice straw cut to about 2 cm in length was added. After stirring, the mixture was sandwiched between 2 pieces of plastic net (mesh size: about 4 mm) in a form of plate. The laminate was placed in an incubator at constant temperature and constant humidity at 28 °C and at a relative humidity of 80% for 7 days so that the enzymatic reaction could proceed. As a result, a light-weight plate material having antimicrobial properties was obtained.

### Example 19: Vessel

A 10 ml reaction mixture (pH 8.5) containing 30 ppm of the freeze-dried product described in Example 1 and 25% of commercially available lignosulfonic acid (LSA) was prepared. This was added to commercially available paper towel (Kimtowel Wiper White, Jujo Kimberly Co., cut to 10 cm x 15 cm in a stack of 8-sheets) so that it could get wetted uniformly. Thereafter, the paper towel was folded to form a box (5 cm x 5 cm x 2.5 cm) as illustrated in Fig. 2 and placed in an incubator at constant temperature and constant humidity at 28 °C and at a relative humidity of 80% for 2 days so that enzymatic macromolecularization reaction could proceed. The dry weight of the box-shaped vessel was measured. Then, the vessel was buried in the soil of open field (5 cm in depth). After it was left to stand for 6 months, the vessel was recovered from the soil and the soil attached thereto was carefully removed before the dry weight of the vessel thus treated was measured. As a result, the vessel showed an about 15% decrease in weight as compared with the weight before it was buried in the soil but it maintained its shape as a vessel. This indicates that the paper towel is useful as a material having biodegradability. For comparison, in the case of the vessel prepared in the same manner as above except that the polyphenol oxidizing enzyme was omitted, it was observed that pieces of paper was peeled off from the laminate after drying, thus indicating a decreased strength. In addition, the decrease in weight after the vessel was buried in the soil is about 80%, and most part of the lignosulfonic acid was eluted and decomposition of the cellulose moiety of the vessel proceeded so that the shape of the vessel was lost.

### Example 20: Deodorant

A reaction mixture (pH 8.5) for treating wood containing 30 ppm of the freeze-dried product described in Example 1, 5% of commercially available lignosulfonic acid (LSA), and 0.2% of (+)-catechin·H₂O was prepared, and Japan cedar wood blocks (2 cm x 0.5 cm x 0.5 cm, butt end: 2 cm x 0.5 cm) were subjected to the reduced pressure impregnation treatment with the reaction mixture thus obtained in the same manner as in Example 3. The wood blocks after the impregnation treatment were placed in an incubation room at 28 °C for 24 hours to allow drying and macromolecularization to proceed.

Then, the wood blocks were charged in a 10 ml test vessel and 30 µl of an aqueous solution of 10% methyl mercaptan and 50% methanol was added along the inner wall without a direct contact with the wood block, and the test vessel was left to stand at 37 °C for 30 minutes. The head space gas of the vessel was extracted through a syringe and subjected to gas chromatography for analyzing the concentration of methyl mercaptan. Also, the concentration of methyl mercaptan was analyzed by repeating the test without the wood blocks and then a proportion of the former concentration to the latter was obtained, from which 1 was subtracted to obtain a deodorization rate. Gas chromatography was run using SUPELCOWAX 10 (0.25 mm ID, 0.25 µm df) (SUPELCO, Inc.) of 30 m long as a column, N₂ (1 ml/minute) as a carrier gas at a column temperature of 60 °C, an inlet temperature of 200 °C and FID as a detector. As a result, the wood blocks described above exhibited a deodorization rate of 90%. For comparison, the deodorization rate was 35% when the non-treated wood blocks were used. This indicates that the treated articles of this invention have effective deodorization effects.

### Example 21: Treatment Agents for Porous Article

10 mg of the freeze-dried product described in Example 1, 10 g of commercially available lignosulfonic acid (LSA) powder, and various chemical agents were mixed well in a mortar to obtain a treatment agent for treating a porous article. If desired, a small amount of sodium carbonate powder was added to the resulting powder and mixed well, so that when a 5% aqueous solution was prepared from the above-described powder the solution could have a pH of 8.0 to 9.0. Then, after the powdery treatment agent was left to stand at room temperature for 2 weeks, 5 g of the powder was dissolved in 100 ml of deionized water to prepare a solution for treating a porous article. Using this solution, Japan cedar wood blocks were subjected to reduced pressure impregnation, drying/macromolecularization reaction, and leaching in the same manner as in Examples 3 and 4. Taking the amounts of lignosulfonic acid and chemical agents injected upon the impregnation treatment as 100%, respectively, the amounts of them which was eluted were compared. The results are shown in Table 12, from which it can be seen that the powdery treatment agent enables effective treatment of porous articles.

Further, 6 g of the powdery treatment agent obtained in the same manner as described above was dissolved in 12 ml of deionized water to obtain a liquid treatment agent for a porous article. The liquid treatment agent was transferred into a 20 ml screw capped test tube. After the test tube stand airtightly at room temperature for 2 weeks, the liquid treatment agent was diluted 10-fold with deionized water to prepare a solution for treating a porous article. Japan cedar wood blocks were subjected to reduced pressure impregnation treatment with the solution thus obtained, drying/macromolecularization reaction, and elution treatment in the same manner as in Examples 3 and 4. The amounts of the lignosulfonic acid which were eluted were calculated and compared taking the amounts of it injected upon the impregnation treatment as 100%. The results are shown in Table 13, from which it can be seen that even with the liquid treatment agent, effective treatment of porous articles is possible.

**Table 12**

| Chemical Agent Used And Its Concentration | Elution Rate (%) | |
|---|---|---|
| | LSA | Chemical Agent |
| - | 0.5 | - |
| CuSO₄.5H₂O(1g) + EDTA (0.234g) | 6.2 | 13.3 (Copper) |
| CuSO₄.5H₂O(2g) + EDA (0.96g) | 0.9 | 3.5 (Copper) |
| CuSO₄.5H₂O(2g) + Glycine (0.6g) | 0.8 | 9.1 (Copper) |
| ZnSO₄ (1.3g)+ EDA(0.96g) | 1.1 | 4.8 (Zinc) |
| NiCl₂ (1.0g)+ EDA(0.96g) | 1.3 | 4.2 (Nickel) |
| Boric acid (2g) | 0.6 | 18.1 (Boron) |
| Hinokitiol (0.2g) | 0.6 | 3.0 |
| Hinokitiol (0.2g)+CuSO₄.5H₂O(0.5g) | 0.8 | 3.5 (H) |
| (+)-Catechin·H₂O (0.2g) | 2.9 | 3.2 |
| Tannic acid (0.1g) | 3.6 | 4.2 |
| o-Phenylenediamine (0.04g) | 1.5 | 0.4 |

**Table 13**

| Chemical Agent Used And Its Concentration | Elution Rate (%) | |
|---|---|---|
| | LSA | Chemical Agent |
| - | 0.5 | - |
| CuSO₄.5H₂O(1g) + EDTA (0.234g) | 6.7 | 13.1 (Copper) |
| CuSO₄.5H₂O(2g) + EDA (0.96g) | 1.7 | 3.8 (Copper) |
| CuSO₄.5H₂O(2g) + Glycine (0.6g) | 0.8 | 10.2 (Copper) |
| ZnSO₄ (1.3g)+ EDA(0.96g) | 1.2 | 5.0 (Zinc) |
| NiCl₂ (1.0g)+ EDA(0.96g) | 1.6 | 4.9 (Nickel) |
| Boric acid (2g) | 0.6 | 17.8 (Boron) |
| Hinokitiol (0.2g) | 0.4 | 2.5 |
| Hinokitiol (0.2g)+CuSO₄.5H₂O(0.5g) | 0.7 | 3.3 (H) |
| (+)-Catechin·H₂O (0.2g) | 2.0 | 3.4 |
| Tannic acid (0.1g) | 3.7 | 4.6 |
| o-Phenylenediamine (0.04g) | 2.1 | 0.8 |

### Example 22: Test of Treated Wood on Termite Preventing Properties

In the same manner as in Example 11, measurement was made of the dry weight of the wood blocks after leaching treatment as obtained in Example 10. The wood blocks were placed on the soil at a distance of about 40 cm around the nest of house termite. After they were left to stand for 2 months, termite preventive properties were observed. Further, the soil on the surface of the wood blocks was removed sufficiently and their dry weight was measured in the same manner as in Example 11. Then, the dry weight thus obtained was compared with the weight of the wood blocks before the placement on the soil, thus calculating a weight reduction rate. The results are shown in Table 14, from which it can be seen that termite preventing properties can be imparted by the method for treating a porous article according to this invention.

**Table 14**

| Conditions for Treating Wood | | | | Weight Reduction Rate(%) |
|---|---|---|---|---|
| LSA | Chemical Agent Used And Its Concentration | Enzyme | pH of Treating Liquid | |
| added | - | not added | 8.5 | 15.3 |
| added | - | added | 8.5 | 5.0 |
| added | 0.04M CuSO₄ + 0.08M EDA | added | 8.1 | 1.0 |
| added | 0.04M ZnSO₄ + 0.08M EDA | added | 8.2 | 1.2 |
| added | 0.04M NiCl₂ + 0.08M EDA | added | 8.2 | 0.8 |
| added | 1000ppm Hinokitiol | added | 8.5 | 2.2 |
| added | 1000ppm Hinokitiol+0.01M CuSO₄ | added | 8.1 | 0.8 |
| Nontreatment | | | | 22.9 |

### Example 23: Copper Retention Rate

Investigation was made on the influences of lignosulfonic acid and oxidoreductases on the rate of copper fixed to wood under various conditions. Aqueous solutions having the compositions listed in table 15 below were prepared and adjusted to pH 8.5 with sodium hydroxide. Those compositions which contained no ethylenediamine (EDA) nor ethanolamine (MEA) were tested at pH 4.5 since copper is deposited as copper hydroxide in alkaline regions. The lignosulfonic acid used was sodium lignosulfonate obtained from Aldrich Chemical Company. As the polyphenol oxidizing enzyme was used the freeze-dried product (10 U/mg) prepared according to the method described in Example 1 in a concentration of 20 ppm.

In this solution were dipped Japan cedar sapwood blocks of 2 cm x 2cm x 1 cm (butt end surface: 2 cm x 2cm, specific gravity: 0.25 to 0.3) which were dried in a forced air circulation drier at 60 °C for 48 hours and the respective solutions were impregnated by treatments under the conditions of at a pressure of reduced pressure to 700 mmHg for 30 minutes and then at atmospheric pressure for 30 minutes. The wood blocks were taken out of the solutions and air-dried at 28 °C for 72 hours and then dried in a forced air circulation drier at 60 °C for 48 hours. After the drying, the wood blocks were submerged in 10 fold volume of deionized water at 25 °C with stirring at 300 rpm for 8 hours for leaching. This operation was repeated three times. The rate of copper fixed to wood was calculated from the sum of the amounts of eluted copper ions by the three elution operations to the total amount of copper ions in the solution impregnated in the wood. The results are shown in Table 15, from which it can be seen that copper fixation rate to wood markedly increases particularly by co-presence of lignosulfonic acid and a oxidoreductase.

**Table 15**

| Chemical Agent Used And Its Concentration | Enzyme | pH of Treating Liquid | Copper Retention Rate(%) |
|---|---|---|---|
| 0.04M CuSO₄ | not added | 4.6 | 21.8 |
| 0.04M CuSO₄ | added | 4.6 | 19.3 |
| 0.04M CuSO₄ + 0.04M EDA | not added | 8.5 | 27.3 |
| 0.04M CuSO₄ + 0.04M EDA | added | 8.5 | 25.9 |
| 0.04M CuSO₄ + 0.04M EDA + 2% LSA | not added | 8.5 | 42.0 |
| 0.04M CuSO₄ + 0.04M EDA + 2% LSA | added | 8.5 | 85.3 |
| 0.04M CuSO₄ + 0.1M MEA | not added | 8.5 | 55.0 |
| 0.04M CuSO₄ + 0.1M MEA | added | 8.5 | 56.2 |
| 0.04M CuSO₄ + 0.1M MEA + 2% LSA | not added | 8.5 | 65.1 |
| 0.04M CuSO₄ + 0.1M MEA + 2% LSA | added | 8.5 | 94.1 |

### INDUSTRIAL APPLICABILITY

By the method for treating a porous article according to this invention which impregnates under pressure or under reduced pressure an enzyme having a polyphenol oxidizing activity in an alkaline pH region, a phenolic compound and/or an aromatic amine compound, and an unsaturated compound or a chemical agent, and allows macromolecularization reaction in the porous article, there can be efficiently obtained porous articles having strength, wear resistance, weatherability, rust-preventing properties, flame resistance, antibacterial properties, antiseptic properties, sterilizing properties, insect-repellent properties, insecticidal properties, antiviral properties, organism-repellent properties, adhesiveness, chemical agent-slow-releasing properties, coloring properties, dimension stability, crack resistance, deodorizing properties, deoxidizing properties, humidity controlling properties, moisture conditioning properties, water repellency, surface smoothness, bioaffinity, ion exchangeability, formaldehyde absorbing properties, chemical agent elution preventing properties, or properties preventing the migration of inorganic compounds onto the surface of the porous article.

The composition for treating the inside of a porous article of this invention is particularly useful when used in the method for treating a porous article according to this invention.

## Claims

1. A composition for treating the inside of a porous article comprising an enzyme having a polyphenol oxidizing activity and a substrate therefor, wherein the composition contains at least one chemical agent selected from a fragrant, a deodorant, a rust preventive, a flame retardant, an antibacterial agent, an antiseptic, a sanitizer, an insect-repellent, an antiviral, agent, and an organism-repellent, the said chemical agent being a solution or powder of a metal salt, a metal compound, or a metal complex, in which one metal selected from copper, arsenic, zinc, chromium, nickel, aluminum, molybdenum, magnesium, or silver.

2. The composition for treating the inside of a porous article, comprising an enzyme having a polyphenol oxidizing activity and a substrate therefor, said composition containing at least one chemical agent selected from a fragrant, a deodorant, a rust preventive, a flame retardant, an antibacterial agent, an antiseptic, a sanitizer, an insect-repellent, an antiviral agent, and an organism-repellent, wherein the chemical agent is a solution or powder of a boron salt, a boron based compound, or a boron-containing complex.

3. The composition for treating the inside of a porous article, comprising an enzyme having a polyphenol oxidizing activity and a substrate therefor, said composition containing at least one chemical agent selected from a fragrant, a deodorant, a rust preventive, a flame retardant, an antibacterial agent, an antiseptic, a sanitizer, an insect-repellent, an antiviral agent, and an organism-repellent, wherein the chemical agent is an aromatic compound or a cyclic compound, having one or more substituent(s) selected from a halogen atom.

4. The composition for treating the inside of a porous article as claimed in any of the claim 1 to 3 wherein the composition contains a phenolic compound and/or an aromatic amine compound as the substrate for the enzyme.

5. The composition for treating the inside of a porous article as claimed in claim 4, wherein the phenolic compound and/or the aromatic amine compound are or is lignin or lignin derivatives.

6. The composition for treating the inside of a porous article as claimed in claim 5, wherein the lignin derivative is lignosulfohic acid or lignosulfonate.

7. The composition for treating the inside of a porous article as claimed in claim 5 or 6, wherein the substrate for the enzyme comprises lignin, lignosulfonic acid, or lignosulfonate obtainable by removing a portion of a water insoluble solid component by centrifugation or filtration.

8. The composition for treating the inside of a porous article as claimed in claim 4, wherein the phenolic compound and/or the aromatic amine compound are or is an aromatic compound having a substituent containing in addition to a hydroxyl group and/or an amino- group, a polyoxyethylene, a polyethyleneimine, or a C1 to C22 saturated or unsaturated alkyl chain as a structural part.

9. The composition for treating the inside of a porous article as claimed in any one of claims 4 to 8, wherein the composition contains an unsaturated fatty acid, an unsaturated alcohol, or an unsaturated alkyl compound.

10. The composition for treating the inside of a porous article as claimed in any of the claims 1 to 9, wherein the enzyme having a polyphenol oxidizing activity is a catechol oxidase, a laccase, a polyphenol oxidase, an ascorbic acid oxidase, or a bilirubin oxidase.

11. The composition for treating the inside of a porous article as claimed in any of the claims 1 to 9, wherein the enzyme having a polyphenol oxidizing activity is a mixture of an enzyme having a peroxidase activity and an oxidase capable of producing hydrogen peroxide.

12. The composition for treating the inside of a porous article as claimed in claim 1, 10 or 11, wherein the enzyme having a polyphehol oxidizing activity is an enzyme obtainable by cultivating genus *Myrothecium.*

13. The composition for treating the inside of a porous article as claimed in any of claims 1 to 12 wherein the enzyme having apoiyphenol oxidizing activity is an enzyme which has an optimum reaction pH on an alkaline side not lower than pH 7.5 when measured of activity using syringaldazine.

14. The composition for treating the inside of a porous article as claimed in any one of claims 1 to 13, wherein the composition is in the form of a high concentration solution to be diluted upon use, or powder or granulated powder to be dissolved upon use.

15. A method for treating a porous article, comprising the steps of impregnating a porous article with a composition for treating the inside of a porous article as claimed in any one of claims 1 to 14 as it is, or in diluted or dissolved state and allowing macromolecularization reaction to occur in the porous article.

16. The method for treating a porous article as claimed in claim 15, wherein the impregnation is performed under pressure and/or under reduced pressure.

17. The method for treating a porous article as claimed in claim 16, wherein pressurization is performed at 1 to 20 alms.

18. The method for treating a porous article as claimed in any one of claims 15 to 17,wherein said at least one chemical, agent is coated or impregnated to the porous article as a pretreatment or posttreatment.

19. The method for treating a porous article as claimed in any one of claims 15 to 18, wherein the porous article is a sintered metal article, a cast article, an alloy, a die-cast article, a ceramic a brick, concrete, wood, processed woody material, chaffs, rush, straw, bamboo, or foamed synthetic resin.

20. The method for treating a porous article as claimed in any one of claims 15 to 19, wherein the phenolic compound and/or the aromatic amine compound in the liquid impregnated to the porous article is in a concentration of 0.1 to 30% by weight.

21. The method for treating a porous article as claimed in any one of claims 15 to 20, wherein an enzyme having a polyphenol oxidizing activity in an alkaline pH region is used.

22. The method for treating a porous article as claimed in any one of claims 15 to 21, wherein the porous article is washed before the macromolecularization reaction in the porous article proceeds sufficiently so that a controlled porosity is obtained.

23. A treated porous article obtainable by a method as claimed in any one of claims 15 to 22.

## Patentansprüche

1. Zusammensetzung für das Behandeln des Inneren eines porösen Gegenstandes, die ein Enzym mit einer Aktivität, durch die Polyphenol oxidiert wird, und ein Substrat dafür enthält, wobei die Zusammensetzung mindestens ein chemisches Mittel enthält, das unter einem Duftstoff, einem Deodorant, einem Rostschutzmittel, einem Flammschutzmittel, einem antibakteriellen Mittel, einem antiseptischen Mittel, einem Sanitärmittel, einem Insektenschutzmittel, einem antiviralen Mittel und einem Mittel zum Vertreiben von Organismen ausgewählt ist, wobei das chemische Mittel eine Lösung oder ein Pulver eines Metallsalzes, einer Metallverbindung oder eines Metallkomplexes ist, wobei das Metall mindestens ein Metall ist, das unter Kupfer, Arsen, Zink, Chrom, Nickel, Aluminium, Molybdän, Magnesium oder Silber ausgewählt ist.

2. Zusammensetzung zum Behandeln des Inneren eines porösen Gegenstandes, die ein Enzym mit einer Aktivität, durch die Polyphenol oxidiert wird, und ein Substrat dafür enthält, wobei die Zusammensetzung mindestens ein chemisches Mittel enthält, das unter einem Duftstoff, einem Deodorant, einem Rostschutzmittel, einem Flammschutzmittel, einem antibakteriellen Mittel, einem antiseptischen Mittel, einem Sanitärmittel, einem Insektenschutzmittel, einem antiviralen Mittel und einem Mittel zum Vertreiben von Organismen ausgewählt ist, wobei das chemische Mittel eine Lösung oder ein Pulver eines Borsalzes, einer Verbindung auf Basis von Bor oder eines Bor enthaltenden Komplexes ist.

3. Zusammensetzung zum Behandeln des Inneren eines porösen Gegenstandes, die ein Enzym mit einer Aktivität, durch die Polyphenol oxidiert wird, und ein Substrat dafür enthält, wobei die Zusammensetzung mindestens ein chemisches Mittel enthält, das unter einem Duftstoff, einem Deodorant, einem Rostschutzmittel, einem Flammschutzmittel, einem antibakteriellen Mittel, einem antiseptischen Mittel, einem Sanitärmittel, einem Insektenschutzmittel, einem antiviralen Mittel und einem Mittel zum Vertreiben von Organismen ausgewählt ist, wobei das chemische Mittel eine aromatische Verbindung oder eine cyclische Verbindung ist, die einen oder mehrere Substituenten aufweist, die unter Halogenatomen ausgewählt sind.

4. Zusammensetzung zum Behandeln des Inneren eines porösen Gegenstandes nach einem der Ansprüche 1 bis 3, wobei die Zusammensetzung eine Phenolverbindung und/oder eine aromatische Aminverbindung als Substrat für das Enzym enthält.

5. Zusammensetzung zum Behandeln des Inneren eines porösen Gegenstandes nach Anspruch 4, wobei die Phenolverbindung und/oder die aromatische Aminverbindung Lignin oder Lignin-Derivate ist bzw. sind.

6. Zusammensetzung zum Behandeln des Inneren eines porösen Gegenstandes nach Anspruch 5, wobei das Ligninderivat Lignosulfonsäure oder Lignosulfonat ist.

7. Zusammensetzung zum Behandeln des Inneren eines porösen Gegenstandes nach Anspruch 5 oder 6, wobei das Substrat für das Enzym Lignin, Lignosulfonsäure oder Lignosulfonat enthält, das durch Entfernen eines Teils einer wasserunlöslichen Feststoffkomponente durch Zentrifugation oder Filtration erhältlich ist.

8. Zusammensetzung zum Behandeln des Inneren eines porösen Gegenstandes nach Anspruch 4, wobei die Phenolverbindung und/oder die aromatische Aminverbindung eine aromatische Verbindung mit einem Substituenten ist bzw. sind, der neben einer Hydroxygruppe und/oder einer Aminogruppe ein Polyoxyethylen, ein Polyethylenimid oder eine gesättigte oder ungesättigte C₁- bis C₂₂-Alkylkette als Strukturteil enthält.

9. Zusammensetzung zum Behandeln des Inneren eines porösen Gegenstandes nach einem der Ansprüche 4 bis 8, wobei die Zusammensetzung eine ungesättigte Fettsäure, einen ungesättigten Alkohol oder eine ungesättigte Alkylverbindung enthält.

10. Zusammensetzung zum Behandeln des Inneren eines porösen Gegenstandes nach einem der Ansprüche 1 bis 9, wobei das Enzym mit einer Aktivität, durch die Polyphenol oxidiert wird, eine Katecholoxidase, eine Laccase, eine Polyphenoloxidase, eine Ascorbinsäureoxidase oder eine Bilirubinoxidase ist.

11. Zusammensetzung zum Behandeln des Inneren eines porösen Gegenstandes nach einem der Ansprüche 1 bis 9, wobei das Enzym mit einer Aktivität, durch die Polyphenol oxidiert wird, ein Gemisch eines Enzyms mit einer Peroxidaseaktivität und einer Oxidase ist, die Wasserstoffperoxid herstellen kann.

12. Zusammensetzung zum Behandeln des Inneren eines porösen Gegenstandes nach Anspruch 1, 10 oder 11, wobei das Enzym mit einer Aktivität, durch die Polyphenol oxidiert wird, ein Enzym ist, das durch Kultivieren eines Mikroorganismus der Gattung Myrothecium erhältlich ist.

13. Zusammensetzung zum Behandeln des Inneren eines porösen Gegenstandes nach einem der Ansprüche 1 bis 12, wobei das Enzym mit einer Aktivität, durch die Polyphenol oxidiert wird, ein Enzym ist, das einen optimalen pH-Wert der Reaktion auf der alkalischen Seite von nicht weniger als 7,5 aufweist, wenn die Aktivität unter Verwendung von Syringaldazine gemessen wird.

14. Zusammensetzung zum Behandeln des Inneren eines porösen Gegenstandes nach einem Ansprüche 1 bis 13, wobei die Zusammensetzung in der Form einer hochkonzentrierten Lösung, die bei Verwendung verdünnt werden muß, oder eines Pulvers oder granulierten Pulvers, das bei Verwendung aufgelöst werden muß, vorliegt.

15. Verfahren zum Behandeln eines porösen Gegenstandes, welches die Stufen umfaßt, bei denen ein poröser Gegenstand mit einer Zusammensetzung zum Behandeln des Inneren eines porösen Gegenstandes nach einem der Ansprüche 1 bis 14 als solcher oder in verdünntem oder gelöstem Zustand imprägniert wird und in dem porösen Gegenstand eine Makromolekularisierungsreaktion zugelassen wird.

16. Verfahren zum Behandeln eines porösen Gegenstandes nach Anspruch 15, wobei die Imprägnierung unter Druck und/oder unter vermindertem Druck durchgeführt wird.

17. Zusammensetzung zum Behandeln eines porösen Gegenstandes nach Anspruch 16, wobei ein Druck von 1 bis 20 Atmosphären ausgeübt wird.

18. Verfahren zum Behandeln eines porösen Gegenstandes nach einem der Ansprüche 15 bis 17, wobei der poröse Gegenstand als Vorbehandlung oder Nachbehandlung mit dem mindestens einen chemischen Mittel überzogen oder imprägniert wird.

19. Verfahren zum Behandeln eines porösen Gegenstandes nach einem der Ansprüche 15 bis 18, wobei der poröse Gegenstand ein gesinterter Metallgegenstand, ein Formgegenstand, eine Legierung, ein spritzgegossener Gegenstand, ein Keramikmaterial, ein Ziegelstein, Beton, Holz, verarbeitetes Holzmaterial, Hächselmaterial, Schilf, Stroh, Bambus oder ein geschäumtes synthetisches Harz ist.

20. Verfahren zum Behandeln eines porösen Gegenstandes nach einem der Ansprüche 15 bis 19, wobei die Phenolverbindung und/oder die aromatische Aminverbindung in der Flüssigkeit, mit der der poröse Gegenstand imprägniert wird, in einer Konzentration von 0,1 bis 30 Gew.-% vorliegt.

21. Verfahren zum Behandeln eines porösen Gegenstandes nach einem der Ansprüche 15 bis 20, wobei ein Enzym mit einer Aktivität, durch die Polyphenol oxidiert wird, in einer alkalischen pH-Region eingesetzt wird.

22. Verfahren zum Behandeln eines porösen Gegenstandes nach einem der Ansprüche 15 bis 21, wobei der poröse Gegenstand gewaschen wird, bevor die Makromolekularisierungsreaktion in dem porösen Gegenstand ausreichend fortgeschritten ist, so daß eine kontrollierte Porosität erzielt wird.

23. Behandelter poröser Gegenstand, erhältlich durch ein Verfahren nach einem der Ansprüche 15 bis 22.

## Revendications

1. Composition de traitement pour l'intérieur d'un article poreux comprenant un enzyme ayant une activité oxydante sur un polyphénol et un substrat pour, **caractérisée en ce que** la composition contient au moins un agent chimique choisi parmi un parfum, un déodorant, un anti-rouille, un retardateur de flamme, un agent antibactérien, un anti-septique, un agent antiviral, et un répulsif d'organisme, ledit agent chimique étant une solution ou une poudre d'un sel métallique, un composé métallique, ou un complexe métallique dans lequel un métal est choisi parmi le cuivre, l'arsenic, le zinc, le chrome, le nickel, l'aluminium, le molybdène, le magnésium ou l'argent.

2. Composition de traitement pour l'intérieur d'un article poreux comprenant un enzyme ayant une activité oxydante sur un polyphénol et un substrat pour, ladite composition contenant au moins un agent chimique choisi parmi un parfum, un déodorant, un anti-rouille, un retardateur de flamme, un agent antibactérien, un anti-septique, un agent antiviral, et un répulsif d'organisme, **caractérisée en ce que** l'agent chimique est une solution ou une poudre d'un sel de bore, un composé à base de bore ou un complexe contenant du bore.

3. Composition de traitement pour l'intérieur d'un article poreux comprenant un enzyme ayant une activité oxydante sur un polyphénol et un substrat pour, ladite composition contenant au moins un agent chimique choisi parmi un parfum, un déodorant, un anti-rouille, un retardateur de flamme, un agent antibactérien, un anti-septique, un agent antiviral, et un répulsif d'organisme, **caractérisée en ce que** l'agent chimique est un composé aromatique ou un composé cyclique, ayant un ou plusieurs substituants choisis comme atome d'halogène.

4. Composition de traitement pour l'intérieur d'un article poreux, selon l'une quelconque des revendications 1 à 3, **caractérisée en ce que** le composé phénolique et/ou le composé amine aromatique sont ou est un substrat pour l'enzyme.

5. Composition de traitement pour l'intérieur d'un article poreux, selon la revendication 4, **caractérisée en ce que** le composé phénolique et/ou le composé amine aromatique sont ou est la lignine ou des dérivés de la lignine.

6. Composition de traitement pour l'intérieur d'un article poreux, selon la revendication 5, **caractérisée en ce que** le dérivé de la lignine est l'acide lignosulfonique ou un lignosulfonate.

7. Composition de traitement pour l'intérieur d'un article poreux, selon la revendication 5 ou 6, **caractérisée en ce que** le substrat pour l'enzyme comprend la lignine, l'acide lignosulfonique, ou un lignosulfonate obtenu par élimination d'une partie d'un composant solide insoluble dans l'eau par centrifugation ou filtration.

8. Composition de traitement pour l'intérieur d'un article poreux, selon la revendication 4, **caractérisée en ce que** le composé phénolique et/ou le composé amine aromatique sont ou est un composé aromatique contenant en plus du groupe hydroxyle et/ou du groupe amine, un groupe polyoxyéthylène, un groupe polyéthylèneimine, ou une chaîne alkyle saturée ou insaturée en C₁ à C₂₂ en tant que partie structurale.

9. Composition de traitement pour l'intérieur d'un article poreux, selon l'une quelconque des revendications 4 à 8, **caractérisée en ce que** la composition contient un acide gras insaturé, un alcool insaturé, ou un composé alkyle insaturé.

10. Composition de traitement pour l'intérieur d'un article poreux, selon l'une quelconque des revendications 1 à 9, **caractérisée en ce que** l'enzyme ayant une activité oxydante sur un polyphénol est une oxydase du catéchol, une laccase, une oxydase de polyphénol, une oxydase d'acide ascorbique ou une oxidase de bilirubine.

11. Composition de traitement pour l'intérieur d'un article poreux, selon l'une quelconque des revendications 1 à 9, **caractérisée en ce que** l'enzyme ayant une activité oxydante sur un polyphénol est un mélange d'un enzyme ayant une activité de peroxydase et une oxydase capable de produire du peroxyde d'hydrogène.

12. Composition de traitement pour l'intérieur d'un article poreux, selon l'une quelconque des revendications 1 à 9, **caractérisée en ce que** l'enzyme ayant une activité oxydante sur un polyphénol peut être obtenu par culture du gène *Myrothecium.*

13. Composition de traitement pour l'intérieur d'un article poreux, selon l'une quelconque des revendications 1 à 12, **caractérisée en ce que** l'enzyme ayant une activité oxydante sur un polyphénol est un enzyme qui a un pH de réaction optimal, dans un domaine alcalin, qui n'est pas inférieur à 7,5 quand l'activité est mesurée en utilisant la syringaldazine.

14. Composition de traitement pour l'intérieur d'un article poreux, selon l'une quelconque des revendications 1 à 13, **caractérisée en ce que** la composition est sous la forme d'une solution à concentration élevée pour être diluée lors de l'utilisation, ou une poudre granulée pour être dissoute lors de l'utilisation.

15. Procédé de traitement d'un article poreux, comprenant les étapes d'imprégnation d'un article poreux avec une composition de traitement pour l'intérieur d'un article poreux, selon l'une quelconque des revendications 1 à 14, telle quelle ou dans un état dilué ou dissous, et permettant à la réaction de macromolécularisation de se produire dans l'article poreux.

16. Procédé de traitement d'un article poreux selon la revendication 15, **caractérisé en ce que** l'imprégnation est effectuée sous pression et/ou sous pression réduite.

17. Procédé de traitement d'un article poreux selon la revendication 16, **caractérisé en ce que** la pressurisation est effectuée à une pression de 1 à 20 atmosphères.

18. Procédé de traitement d'un article poreux selon l'une quelconque des revendications 15 à 17, **caractérisé en ce qu'**au moins un agent chimique est appliqué sur ou imprègne l'article poreux en tant que pré-traitement ou post-traitement.

19. Procédé de traitement d'un article poreux selon l'une quelconque des revendications 15 à 18, **caractérisé en ce que** l'article poreux est un article en métal fritté, une céramique, une brique, du ciment, du bois, un produit ligneux transformé, de la menue paille, du jonc, de la paille, du bambou ou une résine synthétique moussée.

20. Procédé de traitement d'un article poreux selon l'une quelconque des revendications 15 à 19, **caractérisé en ce que** le composé phénolique et/ou le composé amine aromatique, dans le liquide qui imprègne l'article poreux est à une concentration de 0,1 à 30 % en poids.

21. Procédé de traitement d'un article poreux selon l'une quelconque des revendications 15 à 20, **caractérisé en ce que** l'enzyme ayant une activité oxydante sur un polyphénol, dans un domaine de pH alcalin, est utilisé.

22. Procédé de traitement d'un article poreux selon l'une quelconque des revendications 15 à 21, **caractérisé en ce que** l'article poreux est lavé avant que la réaction de macromolécularisation dans l'article poreux se produise suffisamment si bien qu'une porosité réglée est obtenue.

23. Article poreux traité qui peut être obtenu par un procédé selon l'une quelconque des revendications 15 à 22.
